# EUROPEAN PATENT SPECIFICATION

(11) **EP 1 651 663 B1**
(45) Date of publication and mention of the grant of the patent: **17.05.2017**
(21) Application number: 04780644.3
(22) Date of filing: 09.08.2004
(51) Int. Cl.: C07K 16/28, C07K 16/46, C07K 14/00, C07K 16/00, C07K 16/44

(54) **BISPECIFIC ANTIBODIES FOR INDUCING APOPTOSIS OF TUMOR AND DISEASED CELLS**
BISPEZIFISCHE ANTIKÖRPER ZUR INDUKTION DER APOPTOSE IN TUMORZELLEN UND ERKRANKTEN ZELLEN
ANTICORPS BISPECIFIQUES POUR INDUIRE L'APOPTOSE DE CELLULES TUMORALES ET MALADES

(30) Priority: 08.08.2003 US 493365 P
(43) Date of publication of application: 03.05.2006
(73) Proprietor: Immunomedics, Inc., Morris Plains, NJ 07950 (US)
(72) Inventor: CHANG, Chien-Hsing, Morris Plains NJ 07950 (US); GOLDENBERG, David, M., Morris Plains NJ 07950 (US); HANSEN, Hans, J., Morris Plains NJ 07950 (US); HORAK, Eva, Morris Plains NJ 07950 (US); HORAK, Ivan, Morris Plains NJ 07950 (US)
(74) Representative: Bohmann, Armin K.
(86) International application number: PCT/US2004/025840
(87) International publication number: WO 2005/014618

(56) References cited:
- WO-A2-02/08291
- WO-A2-03/068821
- WO-A2-03/074567
- US-A- 5 627 025
- US-A1- 2003 124 058
- US-A1- 2003 219 433
- REFF MITCHELL E ET AL: "Future of monoclonal antibodies in the treatment of hematologic malignancies." CANCER CONTROL : JOURNAL OF THE MOFFITT CANCER CENTER 2002 MAR-APR LNKD- PUBMED:11965235, vol. 9, no. 2, March 2002 (2002-03), pages 152-166, XP002599642 ISSN: 1073-2748
- GHETIE M-A ET AL: "Homodimers but not monomers of Rituxan (chimeric anti-CD20) induce apoptosis in human B-lymphoma cells and synergize with a chemotherapeutic agent and an immunotoxin" BLOOD, AMERICAN SOCIETY OF HEMATOLOGY, US LNKD- DOI:10.1182/BLOOD.V97.5.1392, vol. 97, no. 5, 1 March 2001 (2001-03-01), pages 1392-1398, XP002485216 ISSN: 0006-4971
- LEONARD J P ET AL: "COMBINATION MONOCLONAL ANTIBODY THERAPY FOR LYMPHOMA: TREATMENT WITH EPRATUZUMAB (ANTI-CD22) AND RITUXIMAB (ANTI-CD20) IS WELL TOLERATED" BLOOD, AMERICAN SOCIETY OF HEMATOLOGY, US, vol. 98, no. 11, PART 01, 16 November 2001 (2001-11-16), page 844A, XP009029894 ISSN: 0006-4971
- XIONG ET AL.: 'Effiicient inhibition of human B- cell lymphoma xenografts with an anti- CD 20 X anti- CD 3 bispecific diabody.' CANCER LETTERS . vol. 177, 2002, pages 29 - 39, XP002980486
- DEEG ET AL.: 'Treatment of steropid-refractory acute graftr-versus-host disease with anti- CD 147 monoclonal antibody ABX-CBL.' BLOOD. vol. 89, no. 7, 01 October 2001, pages 2052 - 2058, XP008107633
- D. M. GOLDENBERG ET AL: "Properties and structure-function relationships of veltuzumab (hA20), a humanized anti-CD20 monoclonal antibody", BLOOD, vol. 113, no. 5, 1 January 2009 (2009-01-01), pages 1062-1070, XP055023681, ISSN: 0006-4971, DOI: 10.1182/blood-2008-07-168146

## Description

### FIELD OF THE INVENTION

The present invention is related to a heteroconjugate comprising at least a first binding arm and a second binding arm, wherein the first binding arm comprises an anti-CD20 monoclonal antibody or an antigen-binding fragment thereof and wherein the second binding arm comprises an anti-CD22 monoclonal antibody or antigen-binding fragment thereof, for use in the treatment of a disease.

In particular, the present invention relates to a heteroconjugate that has at least two binding arms, and that inhibits growth and induces apoptosis of a diseased cell without requiring the recuitment of effector cells, wherein the first binding arm possesses a different specificity from the second binding arm and wherein the first and second binding arms need not have apoptotic activity when not conjugated to each other for use in the treatment of a disease.

### BACKGROUND OF THE INVENTION

Various antibodies have been found useful for destroying cancerous cells to which they bind by their ability to (i) fix complement (referred to as complement-dependent cytotoxicity, CDC), (2) recruit effector cells (referred to as antibody-dependent cellular cytotoxicity, ADCC) or (3) induce apoptosis.

For an intact and unconjugated monoclonal antibody (MAb), the *in vivo* anti-tumor activity may result from one or a combination of the above mentioned three principal mechanisms of action. Both CDC and ADCC require the Fc portion of a MAb and the effect of ADCC can be augmented by increasing the binding affinity for FcγR (IgG Fc receptors) on effector cells (Shinkawa, et al., J. Biol. Chem. 278: 3466-3473, 2003; Shields et al., J. Biol. Chem. 277: 26733-26740, 2002; Shields et al., J. Biol. Chem. 276: 6591-6604,2002; Davies et al., Biotechnol. Bioeng. 74: 288-294, 2001; and Umana et al., Nature Biotechnol. 176-180, 1999).

Some MAbs can induce apoptosis via negative signaling (Ghetie et al., Proc. Natl. Acad. Sci. USA 94: 7509-7514, 1997; and U.S. patent No. 6,368,596 B1). Negative signaling, as defined by Ghetie *et al.,* is the inhibition of cell growth by cell cycle arrest or the induction of apoptosis (programmed cell death). Examples of these antibodies include an anti-idiotype antibody described by Levy, R. et al. (J. Natl. Cancer Inst. Monogr. 10: 61-68, 1990) and an anti-CD19 MAb (Hamblin, T.J. et al., Br. J. Cancer 42: 495-502, 1980).

Example of a T cell antibody that modulates negative signalling is an antibody that is directed against a T cell activation-associated antigen, CD147. This antibody has been reported to prevent TCR stimulation-dependent reorganization and clustering of microdomains. The disturbance of the microdomains is followed by a selective inhibition of TCR-mediated T cell proliferation. Staffler, G. et al., J. Immunol. 171(4) 1707-1714, 2003.

The potential of a MAb to induce apoptosis via such signaling mechanism appears to be dependent on several factors, namely, (1) the specific cell surface antigen that it binds; (2) the nature of the cell that expresses such antigen; (3) the strength of the antibody-antigen binding interaction; and (4) the ability of the antibody to crosslink its antigen.

Anti-B-cell MAbs that do not show any measurable apoptotic effect on,their target cells upon binding can reproducibly stimulate cell suicide when sufficiently aggregated with a crosslinking second antibody, as demonstrated for anti-CD19 and anti-CD22 antibodies (Chaouchi et al., J. Immunol. 154: 3096-3104, 1995), anti-CD20 antibodies (Shan et al., Blood 91: 1644-1652, 1998; Pedersen, et al., Blood 99: 1314-1319, 2002; Cardarelli et al., Cancer Immunol. Immunother. 51: 15-24, 2002; and Mimori et al., Leukemia 17: 1164-1174,2003), anti-CD24 antibodies (Suzuki et al., J. Immunol., 166: 5567-5577, 2001) and anti-CAMPATH-1 (CD52) antibodies (Rowan et al., Immunol. 95: 427-36, 1998).

Bivalent MAbs that exert little or no apoptotic effect can become potent anti-tumor agents when they are converted into tetravalent homodimers by chemically crosslinking, as demonstrated by Ghetie et al., Proc. Natl. Acad. Sci. USA 94: 7509-7514, 1997; Ghetie et al., Blood 97: 1392-1398, 2001; and in U.S. patent No. 6,368,596 B1. This activity is dependent upon which cell surface antigen that the MAb binds, and does not require the Fc portion or additional crosslinking by a second antibody. For example, Ghetie *et al.* (1997 and 2001) demonstrated that homodimerized monoclonal antibodies, such as anti-CD19, anti-CD-20, anti-CD21 and anti-CD22, possessed superior anti-growth and apoptosis-inducing properties in several neoplastic B cell lines in vitro than their monomeric counterparts. In addition, treatment with tetravalent F(ab')₂ or IgG homodimers, in contrast with monomers, rendered the tumor cells more sensitive to chemotherapeutic agents and synergized with an anti-CD-22-immunotoxin *in* vitro. As disclosed in U.S. patent No. 6,368,596 B1, the use of heterodimers of IgG or other derivatives has been mentioned, but no example or claim was included for the use of such heterodimers.

Uhr et al., in U.S. patent No. 5,686,072, disclose the significantly enhanced anti-tumor activity of a mixture of anti-CD22 and anti-CD19 immunotoxins in SCID-DAUDI mice having disseminated human DAUDI lymphoma. Inhibition of cell proliferation by anti-CD19 requires crosslinking and is dependent upon the affinity of the CD 19 antibody.

Engineered antibodies, bearing three or more functional antigen binding sites (either as Fab or ScFv), with or without the Fc portion, have also been constructed to mimic homodimers of IgG and shown to induce apoptosis without requiring further crosslinking (Miller et al., J. Immunol. 170: 4854-4861, 2003 and WO 01/77342 A1).

It is noted that a bivalent anti-Her2 MAb (MAb74) has been reported to induce apoptosis without the need for further crosslinking either as a homodimer or through the ligation of a second antibody (U.S. patent No. 6,458,356 B1). An anti-CD20 MAb (tositumomab or B1) can induce apoptosis as a bivalent IgG or F(ab')₂ without the need for further crosslinking (Cardarelli, et al., Cancer Immunol. Immunother. 51: 15-24, 2002).

Nakamura *et al.* have described an anti-CD23 Mab (P5E8) that inhibits IgE production via crosslinking with the Fc domain. (Nakamura T. et al., Int. J. Immunopharmacol, 22(2):131-41, 2000). Heterodimers of p5E8 and an anti-CD20 chimeric antibody (C2B8 or rituxan) were reported to inhibit growth and induce apoptosis of CD20/CD23 positive lymphoma cell lines, DHL-4 and SKW (Reff et al., Cancer Control, 9: 152-166, 2002).

An anti-Dr/CD2 bispecific antibody may cross link T memory cells to antigen presenting cells. This bispecific antibody can kill CD2+ memory T cells in psoriatic patients by the targeting of CD2 with an antibody (MEDI-507) or Fc-CD2 ligand (Amevive) leading to the apoptosis of T cells and does require Fc-crosslinking (via NK cells or macrophages). Zhang, Z. et al., Blood 102(1):284-8, 2003; Branco, L. et al., Transplantation 68(10): 1588-96,1999; and Krueger, G.G. et al., J. Am. Acad. Dermatol. 49(2S): S87-97, 2003.

Bispecific antibodies (BsAbs) are designed to recognize two different antigens with their two binding arms. BsAbs that target tumor antigens and effector molecules have been successfully used for *in vivo* detection and therapy of cancers in both preclinical and clinical studies. For example, the Affinity Enhancement System (AES), which utilizes anti-tumor x anti-hapten BsAbs and bivalent haptens labeled with clinically useful radioisotopes, is very effective in concentrating radioactivity at the target tumor, with very high tumor to nontumor ratios. See U.S. Patent No. 5,256,395. BsAbs that bind to a tumor antigen and a triggering molecule on the cell surface of leukocytes, such as CD16 (FcγRIII) on NK cells, or CD3 on cytotoxic T-cells, can mediate lysis of the target tumor cell. Other applications of BsAbs in cancer therapy included localization of cytotoxic drugs or toxins to tumor cells that express the target antigen. Therapeutic intervention using a cocktail of two MAbs against different antigens to treat cancer has been contemplated and is being evaluated in clinical trials for non-Hodgkin's lymphoma (NHL) using an anti-CD22 humanized IgG1 (hLL2) and Rituximab (an anti-CD20 mouse-human chimeric antibody), with encouraging results. However, the cytotoxic effect of BsAbs that bind to two different antigens on the same target cell has not been demonstrated until the present discovery. The use of bispecific or multispecific constructs derived from the combination of anti-CD19, anti-CD20, and anti-CD22 antibodies for therapy of B-cell malignancies and autoimmune disorders has been proposed in WO 00/67795 A1, WO 00/74718 A1, and U.S. Patent Publication Nos. 20020041847 and 20030133930.

The cytotoxic effect of three humanized antibodies, hLL2 (anti-CD22), hLL1 (anti-CD74) and hA20 (anti-CD20), on three human Burkitt's lymphoma cell lines (Raji, Daudi, and Ramos) has been evaluated under conditions that exclude the possibility of CDC and ADCC (Qu, *et al.,* unpublished results). The results obtained to date indicate that none of the three antibodies when examined as whole IgG in solution exert measurable toxicity. Growth inhibition and apoptosis of all three cell lines were observed for hLL1 and hA20, but not hLL2, in the presence of a crosslinking antibody (goat anti-human Fc). Immobilizing hLL2 on ELISA plates was found to induce growth inhibition and apoptosis of Ramos (the other two cell lines have not been tested); however, immobilizing the other two antibodies under similar conditions did not show any cytotoxicity.

There is a need to provide a therapeutic bispecific binding protein, preferably a heteroconjugate form that does not possess Fc regions, yet which effects growth inhibition and induces apoptosis of diseased cells (e.g., tumor cells or other cells), which even more preferably does not require the recruitment of effector cells. There is a continuing need to provide these types of therapeutic bispecific antibodies that are effective at relatively low doses and have reduced cytotoxicity, increased avidity and good bioavailability.

Reff M.E. et al., (Reff M.E. et al., Cancer Control: Journal of the Moffitt Cancer Center 2002, vol. 9, no.2, March 2002, pp. 152-166) provide a review on improvements in the design of monoclonal antibodies to enhance their effectiveness over the then existing therapeutic monoclonal antibodies then approved for treating hematological malignancies.

International patent application WO 02/08291 is related to a multi-specific reagent comprising at least one first binding site for a cell surface receptor which requires multimer ligand bond for the stimulation thereof, and a second binding site for a target antigen which is expressed on the same cell as the cell surface receptor.

Published US patent application US 2003/124058 discloses a method for treating a B-cell malignancy comprising the step of administering to a subject having a B-cell malignancy a therapeutic composition comprising a pharmaceutically acceptable carrier and at least one naked anti-CD19 antibody or at least one naked anti-CD22 antibody, wherein the at least one naked anti-CD22 antibody is administered parenterally in a dosage of from 20 to 1500 mg per dose.

### SUMMARY OF THE INVENTION

The problem underlying the present invention is solved by the subject matter of the attached independent claims. Preferred embodiments may be taken from the attached dependent claims.

More specifically, the problem underlying the present invention is solved in a first aspect by a heteroconjugate comprising at least a first binding arm and a second binding arm,
wherein said first binding arm comprises an anti-CD20 monoclonal antibody or an antigen-binding fragment thereof,
wherein said second binding arm comprises an anti-CD22 monoclonal antibody or an antigen-binding fragment thereof, and
wherein said heteroconjugate is capable of inducing apoptosis in a Daudi cell when said heteroconjugate is contacted with the Daudi cell for 24 hours,
for use in a method for treatment of a B-cell related lymphoma, chronic lymphocytic leukemia or acute lymphocytic leukemia.

More specifically, the problem underlying the present invention is solved in a second aspect by the use of a heteroconjugate comprising at least a first binding arm and a second binding arm,
wherein said first binding arm comprises an anti-CD20 monoclonal antibody or an antigen-binding fragment thereof,
wherein said second binding arm comprises an anti-CD22 monoclonal antibody or an antigen-binding fragment thereof, and
wherein said heteroconjugate is capable of inducing apoptosis in a Daudi cell when said heteroconjugate is contacted with the Daudi cell for 24 hours,
for the manufacture of a medicament for the treatment of a B-cell related lymphoma, chronic lymphocytic leukemia or acute lymphocytic leukemia.

In an embodiment of the first and second aspect, said anti-CD20 monoclonal antibody or antigen-binding fragment thereof comprises CDRs of the light chain and heavy chains of hA20 antibody, and said anti-CD22 antibody or antigen-binding fragment thereof comprises CDRs of the light chain and heavy chains of hLL-2 antibody.

In an embodiment of the first and second aspect, said anti-CD20 monoclonal antibody and anti-CD22 monoclonal antibody respectively comprise framework sequences of variable regions of light and heavy chains of a human antibody.

In an embodiment of the first and second aspect, said first and second binding arms do not have apoptotic activity when not conjugated to each other.

In an embodiment of the first and second aspect, said heteroconjugate comprises a fusion protein comprising said first and said second binding arm.

In an embodiment of the first and second aspect, said first and said second binding arms are conjugated via a chemical linkage.

In an embodiment of the first and second aspect, said heteroconjugate induces apoptosis in a dose-dependent manner without cross-linking of heteroconjugates to each other.

In an embodiment of the first and second aspect, said heteroconjugate is a multi-specific heteroconjugate that binds to more than two antigens.

In an embodiment of the first and second aspect, said binding arms are human, murine, chimeric, primatized or humanized antibodies or antigen-binding fragments.

In an embodiment of the first and second aspect, said heteroconjugate has a structure selected from the group consisting of IgG x Fab', IgG x sFv, F(ab')₂ x Fab', Fab' x Fab', Fab' x sFv, (sFv x sFv)₂, sFv x sFv, diabody, triabody, tetrabody, and quintabody.

In an embodiment of the first and second aspect, said heteroconjugate is made up of a bispecific antibody or a multispecific antibody having greater than two specific binding proteins.

In an embodiment of the first and second aspect, said bispecific antibody has a bivalent Fab' x Fab' structure.

In an embodiment of the first and second aspect, said heteroconjugate further comprises at least one compound selected from the group consisting of a chelator, a chemotherapeutic agent, an enzyme, a hormone, an immunomodulator, an oligonucleotide, a radionuclide, a boron compound, a photoactive agent and a toxin.

In an embodiment of the first and second aspect, said compound is a chelator selected from the group consisting of DTPA, DOTA, TETA, NOTA and a suitable peptide to which a detectable label or a cytotoxic agent can be conjugated.

In an embodiment of the first and second aspect, said compound is a chemotherapeutic agent selected from the group consisting of anthracyclines, taxanes, nitrogen mustards, ethylenimine derivatives, alkyl sulfonates, nitrosoureas, triazenes; folic acid analogs, pyrimidine analogs, purine analogs, antisense oligonucleotides, antagonists or inhibitors of transcription factors, alkaloids, antibiotics, enzymes, platinum coordination complexes, COX-2 inhibitors, apoptotic agents, thalidomide and its derivatives, substituted urea, methyl hydrazine derivatives, adrenocortical suppressants, or antagonists.

As defined in the claims, there provided a heteroconjugate that contains at least two binding arms and that inhibits growth and induces apoptosis of a diseased cell and does not require recruitment of effector cells for use in the treatment of the indicated diseases. The first binding arm possesses a different specificity from the second binding arm and each of the binding arms need not have apoptotic activity when not conjugated to each other. The binding arms may be an antibody or a fragment thereof. The arms may be linked by chemical conjugation or as fusion proteins. The manner by which the heteroconjugate induces apoptosis is dose-dependent and does not involve the cross-linking of heteroconjugates to each other.

In another embodiment of the invention, the heteroconjugate can be used at a dose range of 0.1 µg/mL to 20 µg/mL *in vitro* and in an adult patient, it can be used at a dose range of about 0.5 mg/kg - 15 mg/kg.

It is disclosed that, the first and second binding arms may either be selected from the group consisting of binding arms targeting CD2, CD3, CD8, CD10, CD19, CD21, CD23, CD24, CD25, CD30, CD33, CD37, CD38, CD40, CD45Ro, CD48, CD52, CD55, CD59, CD70, CD74, CD80, CD86, CD138, CD147, HLA-DR, CEA, CSAp, CA-125, TAG-72, EFGR, HER2, HER3, HER4, IGF-1R, c-Met, PDGFR, MUC1, MUC2, MUC3, MUC4, TNFR1, TNFR2, NGFR, Fas (CD95), DR3, DR4, DR5, DR6, VEGF, PIGF, ED-B fibronectin, tenascin, PSMA, PSA, carbonic anhydrase IX, and IL-6. The first binding arm specifically binds to a human tumor target associated with lymphomas or solid tumors.

Contemplated herein are heteroconjugates that are bispecific antibodies having a bivalent Fab' X Fab' structure. Also embodied in the present inventions are multi-specific heteroconjugates that bind to more than two antigens.

Also disclosed herein is a method of treating a disorder comprising administering to a subject in need thereof a therapeutic effective amount of a heteroconjugate that contains at least two binding arms, where
(i) the first binding arm possesses a different specificity from the second binding arm;
(ii) the heteroconjugate inhibits growth and induces apoptosis of a diseased cell and does not require the recruitment of effector cells; and
(iii) the first and second binding arms need not have apoptotic activity when not conjugated to each other.

Further disclosed is a method for treating a disorder, the improvement comprising inhibiting growth and inducing apoptosis of a diseased cell in a subject comprising administering a heteroconjugate that contains at least two binding arms, where
(i) the first binding arm possesses a different specificity from the second binding arm;
(ii) the heteroconjugate inhibits growth and induces apoptosis of a diseased cell and does not require the recruitment of effector cells; and
(iii) the first and second binding arms need not have apoptotic activity when not conjugated to each other.

The subject may be a human or an animal. The disease can be, for example, a B cell-related disease, a T-cell related disease, an immune dysregulation disease, an acute or chronic inflammatory disease, a solid cancer, a hematopoietic tumor, a metabolic disease, a neurodegenerative disease or an autoimmune disease. Specific examples include a carcinoma, a sarcoma, a glioma, a lymphoma, a leukemia, a myeloma, and a skin cancer. Carcinomas include skin, esophageal, gastric, colonic, rectal, pancreatic, lung, breast, ovarian, urinary bladder, endometrial, cervical, testicular, renal, adrenal and liver cancers. Examplary B-cell related diseases include human and veterinary disease and include an indolent form of B-cell lymphoma, an aggressive form of B-cell lymphoma, a chronic lymphocytic leukemia, an acute lymphocytic leukemia, a Waldenström's macroglobulinemia, a multiple myeloma, and non-Hodgkin's lymphoma. T-cell related diseases include human or veterinary T-cell leukemia, skin psoriasis, psoriatic arthritis or mycosis fungoides.

Other diseases include metabolic diseases such as amyloidosis, neurodegenerative diseases such as Alzheimer's disease, and autoimmune diseases such as acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcalnephritis, erythema nodosurn, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitisubiterans, Sjögren's syndrome, primary biliary cirrhosis, Hashimoto's thyroiditis,thyrotoxicosis, scleroderma, chronic active hepatitis, polymyositis/dermatomyositis, polychondritis, parnphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis, or fibrosing alveolitis.

Other diseases also include: acute or chronic inflammatory diseases such as Crohn's Disease, ulcerative colitis, psoriasis, chronic bronchitis, asthma, emphysema, myositis, or polymyositis; and immune dysregulation diseases such as graft versus host disease, organ graft rejection disease, cachexia, atherosclerosis, and septicemia.

Also disclosed is a pharmaceutical composition comprising pharmaceutical suitable carrier and a therapeutic effective amount of heteroconjugate that contains at least two binding arms, where
(i) the first binding arm possesses a different specificity from the second binding arm;
(ii) the heteroconjugate inhibits growth and induces apoptosis of a diseased cell and does not require the recruitment of effector cells; and
(iii) the first and second binding arms need not have apoptotic activity when not conjugated to each other.

Also disclosed is a method of treating psoriasis comprising administering to a subject in need thereof a therapeutic effective amount of a heteroconjugate that contains at least two binding arms, where
(i) the first binding arm possesses a different specificity from the second binding arm;
(ii) the heteroconjugate inhibits growth and induces apoptosis of a psoriatic cell and does not require the recruitment of effector cells; and
(iii) the first and second binding arms need not have apoptotic activity when not conjugated to each other.

Exemplary heteroconjugates useful for treating psoriasis, according to the present disclosure, are bispecific antibodies that have a bivalent Fab' X Fab' structure. These heteroconjugates include, but are not limited to, anti-CD2 X anti-CD25, anti-CD8 X anti-CD25, and anti-CD2 X anti-CD147.

Another aspect of the present disclosure includes a method of diagnosing a disorder comprising administering to a subject a diagnostic composition comprising a pharmaceutical acceptable carrier and the heteroconjugate, as described above.

### BRIEF DESCRIPTION OF THE DRAWINGS

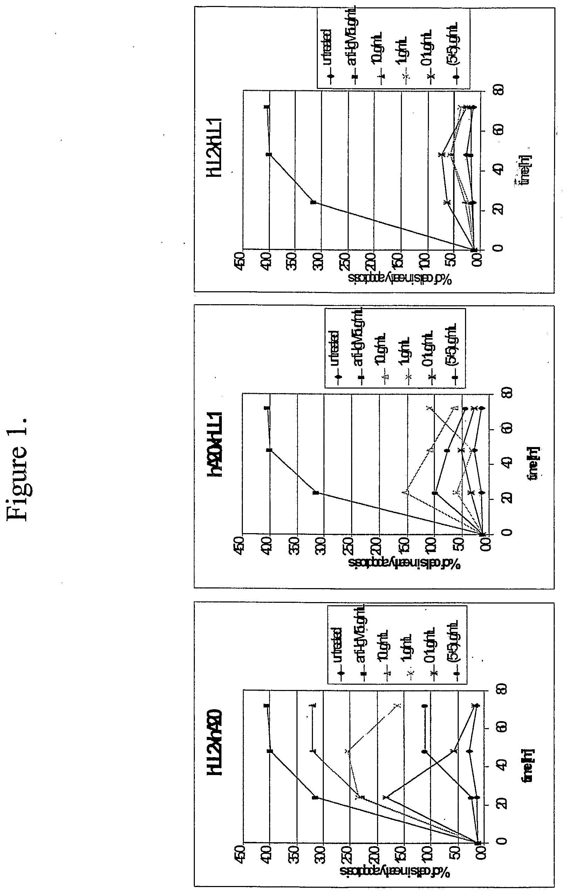

Figure 1 shows the effect of the three BsAbs (hLL2 x hA20, hA20 x hLL1, and hLL2 x hLL1) at indicated concentrations on Daudi to induce apoptosis as determined by the Guava Nexin Assay. Daudi cells (0.8x10⁵) were seeded in 200 µL media into 48-well plates. Tested BsAbs (200 µL) were added to the cells from 2-fold concentrated solutions. Three controls were included: (1) the negative control was provided by cells growing in media only; (2) the positive control for apoptosis was provided by cells incubated with anti-IgM antibody; and (3) the antibody control was provided by cells incubated with the two parental F(ab')₂ which target the same pair of antigens as the test BsAb. Plates were incubated at 37 °C and supplied with 5% CO₂. At the selected time points, analysis was performed to determine the % cells in early apoptosis.

### DETAILED DESCRIPTION OF THE INVENTION

Unless otherwise specified, "a" or "an" means "one or more".

The present invention provides a heteroconjugate as defined in the claims that caused measurable growth inhibition and apoptosis of tumor cells in a dose-dependent manner. This effect can be detected at a concentration as low as 0.1 µg/mL and is comparable to those disclosed in the art.

The present invention also provides the use of a bivalent bispecificantibody, anti-CD22 X anti-CD20, in the form of Fab' x Fab' structure, which was definitively shown to induce apoptosis in target lymphoma cells. The bivalent bispecific antibody differs from the known bispecific antibodies that are capable of inducing tumor apoptosis (e.g., CD19 X CD3 and CD30 X CD16 (NK cells)), in that its anti-tumor growth and anti-apoptotic effects do not require the recruitment of effector cells.

The inventors of the present invention have studied the mechanisms by which several bispecific antibodies, that are in the form of heteroconjugates and that are directed against several B-cell surface antigens, induce growth arrest and apoptosis in tumor cells. More importantly, they have demonstrated the use of a bivalent bispecific anti-CD22 x anti-CD20 antibody (hLL2 x hA20), in an Fab' x Fab' form, is capable of inducing apoptosis to target lymphoma cells. Other bispecific conjugates prepared by either linking hLL2 Fab' with hLL1 Fab' (hLL2 x hLL1) or hA20 Fab' with hLL1 Fab' (hA20 x hLL1) did not inhibit any cytotoxic comparable to hLL2 x hA20.

These bivalent bispecific antibodies can also exert similar apoptotic effects on diseased cells expressing the target antigens. Examples include: B-cells, for example B-cells stimulated in autoimmune diseases; T- cells, and; macrophages and dendritic cells contributing, for example, to acute and chronic inflammation and to immune dysregulation diseases, including graft versus host disease, organ graft rejection disease, cachexia, septicemia, and the like. Atherosclerosis has also been considered to have an underlying inflammatory process. See, for example, Chen et al., Arterioscler Thromb Vasc Biol. Apr;24(4):709-14 Epub Jan 29 2004; Lolis et al., Expert Opin Ther Targets. 7:153-64 (2003); Lue et al., Microbes Inflect. Apr;4:449-60 (2002).

In the description that follows, a number of terms are used and the following definitions are provided to facilitate understanding of the present invention.

The term "apoptosis" refers to programmed cell death as characterized by DNA fragmentation, cell shrinkage, dilation of endoplasmic reticulum, cell fragmentation, and/or formation of membrane vesicles (referred as apoptotic bodies). For example, phosphatidylserine (PS) translocation can be measured by annexin V binding; DNA fragmentation can be evaluated through DNA laddering, propidium iodide staining or TUNEL assay using BrDU labeling, and nuclear/chromatin condensation along with DNA fragmentation can assessed by any increase in hypodiploid cells. Apoptosis can also be measured by using an assay that measures the amount of caspase enzymes in the cells.

"Antibody-dependent cell mediated cytotoxicity" or "ADCC" is a cell-mediated reaction in which nonspecific cytotoxic cells that express Fc receptors (natural killer cells, neutrophils, and macrophages) recognize bound antibody on target cells and subsequently cause lysis of the target cells. The primary cells for mediating ADCC are the natural killer cells (express the FcγRIII only) and monocytes (express FcγRI, FcγRII and FcγRIII). FcR expression on hematopoeitic cells is summarized in Table 3 on page 464 of Ravetch and Kinet, Ann. Rev. Immunol. 9: 457-92,1991.

"Complement-dependent cytotoxicity" or "CDC" refer to the lysing of a target in the presence of complement. The complement activation pathway is initiated by the binding of the first component of the complement system (C1q) to a molecule (*e.g*., an antibody) complexed with a cognate antigen.

The "Fc receptor" and "FcR" is used to describe a receptor that binds to the Fc region of an antibody.

An "effector cell" refers to any type of cell which is capable of carrying out effector cell function(s). It is well known that effector cells having different specialized immune functions can be distinguished from one another on the basis of their differential expression of a wide variety of cell surface antigens, such as many of the antigens described herein to which binding domain polypeptides can specifically bind. Effector cells include any cell that is capable of directly mediating an activity which is a component of immune system function, including cells having such capability naturally or as a result of genetic engineering. An effector cell comprises a cell surface receptor for an immunoglobulin, such as a receptor for an immunoglobulin constant region and including the class of receptors commonly referred to as "Fc receptors" (FcR). Cells that are capable of mediating ADCC are examples of effector cells. Other examples include natural killer (NK) cells, tumor-infiltrating T lymphocytes (TIL), cytotoxic T lymphocytes (CTL), and granulocytic cells such as cells that comprise allergic response mechanisms. Effector cells can also cells of hematopoietic origins including cells at various stages of differentiation within myeloid and lymphoid lineages and which may (but need not) express one or more types of functional cell surface FcR, such as T lymphocytes, B lymphocytes, NK cells, monocytes, macrophages, dendritic cells, neutrophils, basophils, eosinophils, mast cells, platelets, erythrocytes, and precursors, progenitors (e.g., hematopoietic stem cells), quiescent, activated and mature forms of such cells. Other effector cells may include cells of non-hematopoietic origin that are capable of mediating immune functions, for example, endothelial cells, keratinocytes, fibroblasts, osteoclasts, epithelial cells and other cells. Immune effector cells may also include cells that mediate cytotoxic or cytostatic events, or endocytic, phagocytic, or pinocytotic events, or that effect induction of apoptosis, or that effect microbial immunity or neutralization of microbial infection, or cells that mediate allergic, inflammatory, hypersensitivity and/or autoimmune reactions.

The term "heteroconjugate" refers to a conjugate that comprises two or more different species of antibodies. A heteroconjugate of the present invention is capable of inhibiting growth and inducing apoptosis of a diseased cell and comprises at least two binding arms that bind to different target epitopes or that possess different specificities from each other. In other words, the first binding arm would possess a different specificity from the second binding arm. And if unconjugated from each other, the first and second binding arms have substantially no apoptotic or growth inhibiting activity. A heteroconjugate is bi-specific if it recognizes two different target epitopes or two different specificities. A heteroconjugate, according to the present invention can be a bivalent, bispecific construct; a trivalent, bispecific construct; a trivalent, trispecific construct; a tetravalent, bispecific construct; or any other conceivable multivalent multispecific construct, prepared either chemically or recombinantly.

The term "homoconjugate" refers to a conjugate that comprises of a single species of monoclonal antibody and is capable of inhibiting growth and inducing apoptosis of tumor cells, as described in the art.

Exemplary heteroconjugates of the present disclosure include, but are not limited to, anti-CD22 X anti-CD20, anti-CD20 X anti-HLA-DR, anti-CD19 X anti-CD20, anti-CD74 X anti-CD22, anti-CD74 X anti-CD20, and anti-CD20 X anti-CD80, anti-CD2 X anti-CD25, anti-CD8 X anti-CD25, and anti-CD2 X anti-CD147.

An antibody that "inhibits growth" is one that inhibits the growth of diseased cells in vitro and/or in vivo. By inhibiting the growth of diseased cells, the percentage of cells in S phase is reduced. Preferred percentage of growth inhibition by an antibody of the present invention can be greater than 20%, preferably greater than 50% at an antibody concentration of about 0.5 µg/mL - 20 µg/mL *in vitro,* and at a dose in adult patients of about 0.5 mg/kg - 15 mg/kg.

An antibody that "induces apoptosis" is one that induces programmed cell death as established by the above-mentioned characteristics. The cell is a diseased cell which expresses the antigen to which the antibody binds and may be one that overexpresses the antigen. Preferred percentage apoptosis induction by the an antibody of the present invention can be greater than 15%, preferably greater than 50% at an antibody concentration of about 0.5 µg/mL - 20 µg/mL *in vitro,* and at a dose in adult patients of about 0.5 mg/kg - 15 mg/kg.

The hLL-2 antibody is a humanized anti-CD22 antibody prepared by combining the CDR regions of murine LL-2 antibody (mLL-2) with variable region framework sequences obtained from human antibodies. The sequence of the heavy and light chain variable regions of hLL-2 are shown in Figure 1 of U.S. Patent No. 5,789,554. As shown in that figure, the kappa light chain of hLL-2 contains the three light chain CDR regions from mLL-2 and the four framework regions of human antibody REI. The heavy chain of hLL-2 contains the three heavy chain CDRs from mLL-2 combined with three framework regions from human antibody EU, together with a fourth framework region from human antibody NEWM.

Single light chain and two heavy chain variable region sequences encoding the humanized anti-hCD20 (hA20) antibody were designed and constructed, as in U.S. Provisional Application Serial No. 60/356,132, entitled "Anti-CD20 Antibodies And Fusion Proteins Thereof And Methods Of Use", filed February 14, 2002, and U.S. Application Serial No. 10/366,709, filed February 14, 2003 claiming the priority of said provisional application and being published as US 2003-0219433. hA20 contains the V_{H} and V_{K} genes of A20, an anti-CD20 antibody, obtained by RT-PCR using the primer pairs VH1BACK/VH1FOR and VK1BACK/VK1FOR, respectively. Orlandi et al., Proc. Natl. Acad. Sci. USA 86: 3833, 1989. Human REI framework sequences were used for V_{K}, and a combination of EU and NEWM framework sequences were used for V_{H}. There are a number of amino acid changes in each chain outside of the CDR regions when compared to the starting human antibody frameworks. The heavy chain of hA20, hA20V_{H}1, contains nine changes, while hA20V_{H}2 contains three changes from the human EU frameworks. hA20V_{H}2 is preferred because it contains more amino acids from the human antibody framework region than hA20V_{H}1. The light chain of hA20, hA20V*_{K}*, contains seven amino acid changes from the REI framework.

The hLL-1 antibody is a humanized anti-CD74 antibody prepared by combining the CDR regions of murine LL-1 antibody (mLL-1) with variable region framework sequences obtained from human antibodies. The sequence of the heavy and light chain variable regions of hLL-1 are shown in published application No. 20040115193.

An "antibody" as used herein refers to a full-length (i.e., naturally occurring or formed by normal immunoglobulin gene fragment recombinatorial processes) immunoglobulin molecule (*e.g*., an IgG antibody) or an immunologically active (i.e., specifically binding) portion of an immunoglobulin molecule, like an antibody fragment. The term "antibody" also includes "humanized" antibodies and even fully human antibodies that can be produced by phage display, gene and chromosome transfection methods, as well as by other means. This term also includes monoclonal antibodies, polyclonal antibodies, multivalent antibodies, multispecific antibodies (e.g., bispecific antibodies).

An "immunogenic response" or "antigenic response" is one that results in the production of antibodies directed to a compound after the appropriate cells have been contacted therewith. The compound that is used to elicit an immunogenic response is referred to as an immunogen or antigen. The antibodies produced in the immunogenic response specifically bind the immunogen used to elicit the response.

The compound that is used to elicit an immunogenic response is referred to as an immunogen or antigen. An "epitope" or "antigenic determinant" is an area on the surface of an immunogen that stimulates a specific immune response directed to the epitope. In proteins, particularly denatured proteins, an epitope is typically defined and represented by a contiguous amino acid sequence. However, in the case of nondenatured proteins, epitopes also include structures, such as active sites, that are formed by the three-dimensional folding of a protein in a manner such that amino acids from separate portions of the amino acid sequence of the protein are brought into close physical contact with each other.

A "hapten" is a small molecule that cannot provoke an immune response unless first bound to an immunogenic carrier molecule. Although a hapten cannot itself provoke an immune response, it is specifically bound by antibodies generated during an immunogenic response to the hapten-carrier conjugate.

Naturally occurring (wild type) antibody molecules are Y-shaped molecules consisting of four polypeptide chains, two identical heavy chains and two identical light chains, which are covalently linked together by disulfide bonds. Both types of polypeptide chains have constant regions, which do not vary or vary minimally among antibodies of the same class (*i.e.,* IgA, IgM, *etc.*), and variable regions. The variable regions are unique to a particular antibody and comprise a recognition element for an epitope. The carboxy-terminal regions of both heavy and light chains are conserved in sequence and are called the constant regions (also known as C-domains). The amino-terminal regions (also known as V-domains) are variable in sequence and are responsible for antibody specificity. The antibody specifically recognizes and binds to an antigen mainly through six short complementarity-determining regions (CDRs) located in their V-domains.

Each light chain of an antibody is associated with one heavy chain, and the two chains are linked by a disulfide bridge formed between cysteine residues in the carboxy-terminal region of each chain, which is distal from the amino terminal region of each chain that constitutes its portion of the antigen binding domain. Antibody molecules are further stabilized by disulfide bridges between the two heavy chains in an area known as the hinge region, at locations nearer the carboxy terminus of the heavy chains than the locations where the disulfide bridges between the heavy and light chains are made. The hinge region also provides flexibility for the antigen-binding portions of an antibody.

An antibody's specificity is determined by the variable regions located in the amino terminal regions of the light and heavy chains. The variable regions of a light chain and associated heavy chain form an "antigen binding domain" that recognizes a specific epitope; an antibody thus has two antigen binding domains. The antigen binding domains in a wildtype antibody are directed to the same epitope of an immunogenic protein, and a single wildtype antibody is thus capable of binding two molecules of the immunogenic protein at the same time. Thus, a wildtype antibody is monospecific *(i.e.,* directed to a unique antigen) and divalent *(i.e.,* capable of binding two molecules of antigen).

"Polyclonal antibodies" are generated in an immunogenic response to a protein having many epitopes. A composition (*e.g*., serum) of polyclonal antibodies thus includes a variety of different antibodies directed to the same and to different epitopes within the protein. Methods for producing polyclonal antibodies are known in the art (see, *e.g.,* Cooper et al., Section III of Chapter 11 in: Short Protocols in Molecular Biology, 2nd Ed., Ausubel et al., eds., John Wiley and Sons, New York, 1992, pages 11-37 to 11-41).

"Antipeptide antibodies" (also known as "monospecific antibodies") are generated in a humoral response to a short (typically, 5 to 20 amino acids) immunogenic polypeptide that corresponds to a few (preferably one) isolated epitopes of the protein from which it is derived. A plurality of antipeptide antibodies includes a variety of different antibodies directed to a specific portion of the protein, i.e., to an amino acid sequence that contains at least one, preferably only one, epitope. Methods for producing antipeptide antibodies are known in the art (see, *e.g.,* Cooper et al., Section III of Chapter 11 in: Short Protocols in Molecular Biology, 2nd Ed., Ausubel et al., eds., John Wiley and Sons, New York, 1992, pages 11-42 to 11-46).

A "monoclonal antibody" is a specific antibody that recognizes a single specific epitope of an immunogenic protein. In a plurality of a monoclonal antibody, each antibody molecule is identical to the others in the plurality. In order to isolate a monoclonal antibody, a clonal cell line that expresses, displays and/or secretes a particular monoclonal antibody is first identified; this clonal cell line can be used in one method of producing the antibodies of the invention. Methods for the preparation of clonal cell lines and of monoclonal antibodies expressed thereby are known in the art (see, for example, Fuller et al., Section II of Chapter 11 in: Short Protocols in Molecular Biology, 2nd Ed., Ausubel et al., eds., John Wiley and Sons, New York, 1992, pages 11-22 to 11-11-36).

A "naked antibody" is an intact antibody molecule that contains no further modifications such as conjugation with a toxin, or with a chelate for binding to a radionuclide. The Fc portion of the naked antibody provides effector functions, such as complement fixation and ADCC (antibody dependent cell cytotoxicity), which set mechanisms into action that may result in cell lysis. See, *e.g*., Markrides, Therapeutic inhibition of the complement system, Pharmacol. Rev. 50:59-87, 1998. In some systems, it appears that the therapeutic action of an antibody depends upon the effector functions of the Fc region (see, *e.g.,* Golay et al., Biologic response of B lymphoma cells to anti-CD20 monoclonal antibody rituximab in vitro: CD55 and CD59 regulate complement-mediated cell lysis, Blood 95: 3900-3908, 2000).

However, it is possible that the Fc portion is not required for therapeutic function in every instance, as other mechanisms, such as apoptosis, can come into play. Moreover, the Fc region may be deleterious in some applications as antibodies comprising an Fc region are taken up by Fc receptor-bearing cells, thereby reducing the amount of thereapeutic antibody taken up by targeted cells or incurring toxicity to non-target cells. Vaswani and Hamilton, Humanized antibodies as potential therapeutic drugs. Ann. Allergy Asthma Immunol. 81: 105-119, 1998.

An "antibody fragment" is a portion of an intact antibody such as F(ab')₂, F(ab)₂, Fab', Fab, Fv, sFv and the like. Regardless of structure, an antibody fragment binds with the same antigen that is recognized by the full-length antibody. For example, an anti-CD20 monoclonal antibody fragment binds with an epitope of CD20. The term "antibody fragment" also includes any synthetic or genetically engineered protein that acts like an antibody by binding to a specific antigen to form a complex. For example, antibody fragments include isolated fragments consisting of the variable regions, such as the "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

Antibody fragments produced by limited proteolysis of wildtype antibodies are called proteolytic antibody fragments. These include, but are not limited to, the following:
"F(ab')₂ fragments" are released from an antibody by limited exposure of the antibody to a proteolytic enzyme, *e.g.*, pepsin or ficin. An F(ab')₂ fragment comprises two "arms," each of which comprises a variable region that is directed to and specifically binds a common antigen. The two Fab' molecules are joined by interchain disulfide bonds in the hinge regions of the heavy chains; the Fab' molecules may be directed toward the same (bivalent) or different (bispecific) epitopes.

"Fab' fragments" contain a single anti-binding domain comprising an Fab and an additional portion of the heavy chain through the hinge region.

"Fab'-SH fragments" are typically produced from F(ab')₂ fragments, which are held together by disulfide bond(s) between the H chains in an F(ab')₂ fragment. Treatment with a mild reducing agent such as, by way of non-limiting example, beta-mercaptoethylamine, breaks the disulfide bond(s), and two Fab' fragments are released from one F(ab')₂ fragment. Fab'-SH fragments are monovalent and monospecific.

"Fab fragments" (*i.e.,* an antibody fragment that contains the antigen-binding domain and comprises a light chain and part of a heavy chain bridged by a disulfide bond) are produced by papain digestion of intact antibodies. A convenient method is to use papain immobilized on a resin so that the enzyme can be easily removed and the digestion terminated. Fab fragments do not have the disulfide bond(s) between the H chains present in an F(ab')₂ fragment.

"Single-chain antibodies" are one type of antibody fragment. The term single chain antibody is often abbreviated as "scFv" or "sFv." These antibody fragments are produced using molecular genetics and recombinant DNA technology. A single-chain antibody consists of a polypeptide chain that comprises both a V_{H} and a V_{L} domains which interact to form an antigen-binding site. The VH and VL domains are usually linked by a peptide of 10 to 25 amino acid residues.

The term "single-chain antibody" further includes but is not limited to a disulfide-linked Fv (dsFv) in which two single-chain antibodies (each of which may be directed to a different epitope) linked together by a disulfide bond; a bispecific sFv in which two discrete scFvs of different specificity is connected with a peptide linker ; a diabody (a dimerized sFv formed when the V_{H} domain of a first sFv assembles with the V_{L} domain of a second sFv and the V_{L} domain of the first sFv assembles with the V_{H} domain of the second sFv; the two antigen-binding regions of the diabody may be directed towards the same or different epitopes); and a triabody (a trimerized sFv, formed in a manner similar to a diabody, but in which three antigen-binding domains are created in a single complex; the three antigen binding domains may be directed towards the same or different epitopes).

"Complementary determining region peptides" or "CDR peptides" are another form of an antibody fragment. A CDR peptide (also known as "minimal recognition unit") is a peptide corresponding to a single complementarity-determining region (CDR), and can be prepared by constructing genes encoding the CDR of an antibody of interest. Such genes are prepared, for example, by using the polymerase chain reaction to synthesize the variable region from RNA of antibody-producing cells. See, for example, Larrick et al., Methods: A Companion to Methods in Enzymology 2:106, 1991.

In "cysteine-modified antibodies," a cysteine amino acid is inserted or substituted on the surface of antibody by genetic manipulation and used to conjugate the antibody to another molecule via, *e.g.,* a disulfide bridge. Cysteine substitutions or insertions for antibodies have been described (see U.S. Patent No. 5,219,996). Methods for introducing Cys residues into the constant region of the IgG antibodies for use in site-specific conjugation of antibodies are described by Stimmel et al. (J. Biol. Chem 275:330445-30450,2000).

A "chimeric antibody" is a recombinant protein that contains the variable domains including the complementarity determining regions (CDRs) of an antibody derived from one species, preferably a rodent antibody, while the constant domains of the antibody molecule are derived from those of a human antibody. For veterinary applications, the constant domains of the chimeric antibody may be derived from that of other species, such as a cat or dog.

A chimeric Ab is constructed by ligating the cDNA fragment encoding the mouse light variable and heavy variable domains to fragment encoding the C domains from a human antibody. Because the C domains do not contribute to antigen binding, the chimeric antibody will retain the same antigen specificity as the original mouse Ab but will be closer to human antibodies in sequence. Chimeric Abs still contain some mouse sequences, however, and may still be immunogenic. A humanized Ab contains only those mouse amino acids necessary to recognize the antigen. This product is constructed by building into a human antibody framework the amino acids from mouse complementarity determining regions.

A "humanized antibody" is a recombinant protein in which the CDRs from an antibody from one species; *e.g*., a rodent antibody, are transferred from the heavy and light variable chains of the rodent antibody into human heavy and light variable domains. The constant domains of the antibody molecule are derived from those of a human antibody. See Gussow and Seemann, Humanization of monoclonal antibodies, Method Enzymol. 203:99-121, 1991 and Vaswani and Hamilton, Ann. Allergy Asthma Immunol. 81:105-119,1998.

A "human antibody" is an antibody obtained from transgenic mice that have been "engineered" to produce specific human antibodies in response to antigenic challenge. In this technique, elements of the human heavy and light chain loci are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7: 13, 1994; Lonberg et al., Nature 368: 856, 1994; and Taylor et al., Int. Immun. 6: 579, 1994. A fully human antibody also can be constructed by genetic or chromosomal transfection methods, as well as phage display technology, all of which are known in the art. See for example, McCafferty et al., Nature 348:552-553, 1990, for the production of human antibodies and fragments thereof *in vitro,* from immunoglobulin variable domain gene repertoires from unimmunized donors. In this technique, antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see *e.g*. Johnson and Chiswell, Current Opinion in Structural Biology 3:5564-571, 1993.

Human antibodies may also be generated by *in vitro* activated B cells. See U.S. Patent Nos. 5,567,610 and 5,229,275.

A "therapeutic agent" is a molecule or atom which is administered separately, concurrently or sequentially with an antibody moiety or conjugated to an antibody moiety, i.e., antibody or antibody fragment, or a subfragment, and is useful in the treatment of a disease. Examples of therapeutic agents include antibodies, antibody fragments, drugs, toxins, enzymes, nucleases, hormones, immunomodulators, oligonucleotides, chelators, boron compounds, photoactive agents or dyes and radioisotopes.

A "diagnostic/detection agent" is a molecule or atom which is administered conjugated to an antibody moiety, i.e., antibody or antibody fragment, or subfragment, and is useful in diagnosing a disease by locating the cells containing the antigen. Useful diagnostic/detection agents include, but are not limited to, radioisotopes, dyes (such as with the biotin-streptavidin complex), contrast agents, fluorescent compounds or molecules and enhancing agents (*e.g.* paramagnetic ions) for magnetic resonance imaging (MRI). U.S. Patent No. 6,331,175 describes MRI technique and the preparation of antibodies conjugated to a MRI enhancing agent. Preferably, the diagnostic/detection agents are selected from the group consisting of radioisotopes, enhancing agents for use in magnetic resonance imaging, and fluorescent compounds. In order to load an antibody component with radioactive metals or paramagnetic ions, it may be necessary to react it with a reagent having a long tail to which are attached a multiplicity of chelating groups for binding the ions. Such a tail can be a polymer such as a polylysine, polysaccharide, or other derivatized or derivatizable chain having pendant groups to which can be bound chelating groups such as, *e.g*., ethylenediaminetetraacetic acid (EDTA), diethylenetriaminepentaacetic acid (DTPA), porphyrins, polyamines, crown ethers, bis-thiosemicarbazones, polyoximes, and like groups known to be useful for this purpose. Chelates are coupled to the antibodies using standard chemistries. The chelate is normally linked to the antibody by a group, which enables formation of a bond to the molecule with minimal loss of immunoreactivity and minimal aggregation and/or internal cross-linking. Other, more unusual, methods and reagents for conjugating chelates to antibodies are disclosed in U.S. Patent 4,824,659 to Hawthorne, entitled "Antibody Conjugates", issued April 25, 1989. Particularly useful metal-chelate combinations include 2-benzyl-DTPA and its monomethyl and cyclohexyl analogs, used with diagnostic isotopes in the general energy range of 60 to 4,000 keV, such as ¹²⁵I, ¹³¹I, ¹²³I, ¹²⁴I, ⁶²Cu, ⁶⁴Cu, ¹⁸F, ¹¹¹In, ⁶⁷Ga, ⁶⁸Ga, ^{99m}Tc, ^{94m}Tc, ¹¹C, ¹³N, ¹⁵O, ⁷⁶Br, ⁹⁷Zr, for radio-imaging. The same chelates, when complexed with nonradioactive metals, such as manganese, iron and gadolinium are useful for MRI, when used along with the antibodies of the invention. Macrocyclic chelates such as NOTA, DOTA, and TETA are of use with a variety of metals and radiometals, most particularly with radionuclides of gallium, yttrium and copper, respectively. Such metal-chelate complexes can be made very stable by tailoring the ring size to the metal of interest. Other ring-type chelates such as macrocyclic polyethers, which are of interest for stably binding nuclides, such as ²²³Ra for RAIT, are encompassed by the invention.

An "immunoconjugate" is an antibody, fusion protein, or fragment thereof conjugated to at least one therapeutic and/or diagnostic/detection agent. The diagnostic/detection agent can comprise a radionuclide or non-radionuclide, a contrast agent (such as for magnetic resonance imaging, computed tomography or ultrasound), and the radionuclide can be a gamma-, beta-, alpha-, Auger electron-, or positron-emitting isotope.

An "expression vector" is a DNA molecule comprising a gene that is expressed in a host cell. Typically, gene expression is placed under the control of certain regulatory elements, including constitutive or inducible promoters, tissue-specific regulatory elements and enhancers. Such a gene is said to be "operably linked to" the regulatory elements.

A "recombinant host" may be any prokaryotic or eukaryotic cell that contains either a cloning vector or expression vector. This term also includes those prokaryotic or eukaryotic cells, as well as transgenic animals, that have been genetically engineered to contain the cloned gene(s) in the chromosome or genome of the host cell or cells of the host cells. Suitable mammalian host cells include myeloma cells, such as Sp2/0-Agl4 cells, and NS0 cells, as well as Chinese Hamster Ovary (CHO) cells, hybridoma cell lines and other mammalian host cell useful for expressing antibodies. Also particularly useful to express MAbs and other fusion proteins, is a human cell line, PER.C6 disclosed in WO 0063403 A2, which produces 2 to 200-fold more recombinant protein as compared to conventional mammalian cell lines, such as CHO, COS, Vero, Hela, BHK and SP2- cell lines. Special transgenic animals with a modified immune system are particularly useful for making fully human antibodies.

As used herein, the term "antibody fusion protein" is a recombinantly produced antigen-binding molecule in which two or more of the same or different natural antibody, single-chain antibody or antibody fragment segments with the same or different specificities are linked. Valency of the fusion protein indicates the total number of binding arms or sites the fusion protein has to an antigen or epitope; i.e., monovalent, bivalent, trivalent or multivalent. The multivalency of the antibody fusion protein means that it can take advantage of multiple interactions in binding to an antigen, thus increasing the avidity of binding to the antigen. Specificity indicates how many antigens or epitopes an antibody fusion protein is able to bind; i.e., monospecific, bispecific, trispecific, multispecific. Using these definitions, a natural antibody, *e.g*., an IgG, is bivalent because it has two binding arms but is monospecific because it binds to one antigen. Monospecific, multivalent fusion proteins have more than one binding site for an epitope but only bind with the same epitope on the same antigen, for example a diabody with two binding sites reactive with the same antigen. The fusion protein may comprise a multivalent or multispecific combination of different antibody components or multiple copies of the same antibody component. The fusion protein may additionally comprise a therapeutic agent. Examples of therapeutic agents suitable for such fusion proteins include immunomodulators ("antibody-immunomodulator fusion protein") and toxins ("antibody-toxin fusion protein"). One preferred toxin comprises a ribonuclease (RNase), preferably a recombinant RNase.

A "multispecific antibody" is an antibody that can bind simultaneously to at least two targets that are of different structure, *e.g*., two different antigens, two different epitopes on the same antigen, or a hapten and/or an antigen or epitope. One specificity would be for a B-cell, T-cell, myeloid-, plasma-, and mast-cell antigen or epitope. Another specificity could be to a different antigen on the same cell type, such as CD20, CD19, CD21, CD23, CD46, CD80, HLA-DR, CD74, and CD22 on B-cells. One specificity could be for a colonic tumor cell, such as against one of the following antigens: CEA, TAG-72, MUC1, CSAP, VEGF, EGFR; the other specificity could be, for example, for a different antigen of this sample list. A "multivalent" antibody has multiple binding arms and can be mono-specific or multi-specific depending on the sites targeted by the binding arms. Multispecific, multivalent antibodies are constructs that have more than one binding site, and the binding sites are of different specificity. For example, a bispecific diabody, where one binding site reacts with one antigen and the other with the another antigen.

Multivalent, multispecific antibody derivatives can be prepared by a variety of conventional procedures, ranging from glutaraldehyde linkage to more specific linkages between functional groups. The antibodies and/or antibody fragments are preferably covalently bound to one another, directly or through a linker moiety, through one or more functional groups on the antibody or fragment, *e.g*., amine, carboxyl, phenyl, thiol, or hydroxyl groups. Various conventional linkers in addition to glutaraldehyde can be used, *e.g*., disiocyanates, diiosothiocyanates, bis(hydroxysuccinimide) esters, carbodiimides, maleirmidehydroxy-succinimde esters, and the like. The optimal length of the linker may vary according to the type of target cell. The most efficacious linker size can be determined empirically by testing (and ensuring) reactivity to both target and Ii. Such immunochemical techniques are well known.

A simple method to produce multivalent antibodies is to mix the antibodies or fragments in the presence of glutaraldehyde. The initial Schiff base linkages can be stabilized, *e.g*., by borohydride reduction to secondary amines. A diiosothiocyanate or carbodiimide can be used in place of glutaraldehyde as a non-site-specific linker.

The simplest form of a multivalent, multispecific antibody is a bispecific antibody. Bispecific antibodies can be made by a variety of conventional methods, *e*.*g*., disulfide cleavage and reformation of mixtures of whole IgG or, preferably F(ab')₂ fragments, fusions of more than one hybridoma to form polyomas that produce antibodies having more than one specificity, and by genetic engineering. Bispecific antibodies have been prepared by oxidative cleavage of Fab' fragments resulting from reductive cleavage of different antibodies. This is advantageously carried out by mixing two different F(ab')₂ fragments produced by pepsin digestion of two different antibodies, reductive cleavage to form a mixture of Fab' fragments, followed by oxidative reformation of the disulfide linkages to produce a mixture of F(ab')₂ fragments including bispecific antibodies containing a Fab' portion specific to each of the original epitopes (i.e., target and Ii). General techniques for the preparation of multivalent antibodies may be found, for example, in Nisonhoff et al., Arch Biochem. Biophys. 93: 470, 1961; Hammerling et al., J. Exp. Med. 128: 1461, 1968; and U.S. patent No. 4,331,647.

More selective linkage can be achieved by using a heterobifunctional linker such as maleimide-hydroxysuccinimide ester. Reaction of the ester with an antibody or fragment will derivatize amine groups on the antibody or fragment, and the derivative can then be reacted with, *e.g*., an antibody Fab fragment having free sulfhydryl groups (or, a larger fragment or intact antibody with sulfhydryl groups appended thereto by, *e.g*., Traut's Reagent). Such a linker is less likely to crosslink groups in the same antibody and improves the selectivity of the linkage.

It is advantageous to link the antibodies or fragments at sites remote from the antigen binding sites. This can be accomplished by, *e.g*., linkage to cleaved interchain sulfydryl groups, as noted above. Another method involves reacting an antibody having an oxidized carbohydrate portion with another antibody which has at lease one free amine function. This results in an initial Schiff base (imine) linkage, which is preferably stabilized by reduction to a secondary amine, *e.g*., by borohydride reduction, to form the final product. Such site-specific linkages are disclosed, for small molecules, in U.S. patent No. 4,671,958, and for larger addends in U.S. patent No. 4,699,784.

The interchain disulfide bridges of the an F(ab')₂ fragment having target specificity are gently reduced with cysteine, taking care to avoid light-heavy chain linkage, to form Fab'-SH fragments. The SH group(s) is(are) activated with an excess of bis-maleimide linker (1,1'-(methylenedi-4,1-phenylene)bis-malemide).

Techniques for producing bispecific antibodies via quadromas are described, for example, by Milstein et al., Nature 305: 537, 1983; and Pohl et al., Int. J. Cancer 54:413, 1993.

Finally, such bispecific antibodies can be produced by genetic engineering. For example, plasmids containing DNA coding for variable domains of an anti-target MAb can be introduced into hybridomas that secrete the antibodies of interest General techniques for producing bispecific antibodies by genetic engineering are described, for example, by Songsivilai et al., Biochem. Biophys. Res. Commun. 164: 271, 1989; Traunecker et al., EMBO J. 10: 3655, 1991; and Weiner et al., J. Immunol. 147: 4035, 1991.

A higher order multivalent, multispecific molecule can be obtained by adding various antibody components to a bispecific antibody, produced as above. For example, a bispecific antibody can be reacted with 2-iminothiolane to introduce one or more sulfhydryl groups for use in coupling the bispecific antibody to an further antibody derivative that binds an the same or a different epitope of the target antigen, using the bis-maleimide activation procedure described above. These techniques for producing multivalent antibodies are well known to those of skill in the art. See, for example, U.S. Pat. No. 4,925,648, and Goldenberg, International Publication No. WO 92/19273.

A "bispecific antibody" is an antibody that can bind simultaneously to two targets which are of different structure. Bispecific antibodies (bsAb) and bispecific antibody fragments (bsFab) have at least one arm that specifically binds to, for example, a B-cell, T-cell, myeloid-, plasma-, and mast-cell antigen or epitope and at least one other arm that specifically binds to a targetable conjugate that bears a therapeutic or diagnostic/detection agent. A variety of bispecific fusion proteins can be produced using molecular engineering. In one form, the bispecific fusion protein is monovalent, consisting of, for example, a scFv with a single binding site for one antigen and a Fab fragment with a single binding site for a second antigen. In another form, the bispecific fusion protein is divalent, consisting of, for example, an IgG with a binding site for one antigen and two scFv with two binding sites for a second antigen.

Bispecific antibodies (bsAbs) have been used in pretargeting and therapeutic purposes. For additional background on the many uses of bispecific antibodies, see U.S. patent application publication Nos. 2002/0006379A1, 2003/011333 A1 and 2003/0133930 A1, and 2003/0103982 A1; U.S. patent Nos. 6,183,744 B1 and 6,458,933 B1; and WO 99/66951.

The bsAbs can be prepared by techniques known in the art, for example, an anti-CD22 tumor Ab and an anti-CD20 are both separately digested with pepsin to their respective F(ab')₂s. The anti-CD22-Ab-F(ab')₂ is reduced with cysteine to generate Fab' monomeric units which are further reacted with the cross-linker bis(maleimido) hexane to produce Fab'-maleimide moieties. The anti-CD20 Ab-F(ab')₂ is reduced with cysteine and the purified, recovered anti-CD20 Fab'-SH reacted with the anti-CD22-Fab'-maleimide to generate the Fab' x Fab' bi-specific Ab. Alternatively, the anti-CD20 Fab'-SH fragment may be coupled with the anti-CD22 F(ab')₂ to generate a F(ab')₂ X Fab' construct, or with anti-CD22 IgG to generate an IgG x Fab' bi-specific construct. In one embodiment, the IgG x Fab' construct can be prepared in a site-specific manner by attaching the anti-CD20 Fab' thiol group to anti-CD22 IgG heavy-chain carbohydrate which has been periodate-oxidized, and subsequently activated by reaction with a commercially available hydrazide-maleimide cross-linker. The component Abs used can be chimerized or humanized by known techniques. A chimeric antibody is a recombinant protein that contains the variable domains and complementary determining regions derived from a rodent antibody, while the remainder of the antibody molecule is derived from a human antibody. Humanized antibodies are recombinant proteins in which murine complementarity determining regions of a monoclonal antibody have been transferred from heavy and light variable chains of the murine immunoglobulin into a human variable domain.

Antibodies against CD8, CD25 and CD147 can be combined with anti-CD2 antibody to produce bispecific antibodies for targeting memory T cells, with the potential to induce apoptosis. CD-147 is described by Staffler, G. et al., J. Immunol. 171(4) 1707-1714, 2003.

Other recent methods for producing bsAbs include engineered recombinant Abs which have additional cysteine residues so that they crosslink more strongly than the more common immunoglobulin isotypes. See, *e.g.,* FitzGerald et al., Protein Eng. 10(10): 1221-1225, 1997. Another approach is to engineer recombinant fusion proteins linking two or more different single-chain antibody or antibody fragment segments with the needed dual specificities. See, *e.g.,* Coloma et al., Nature Biotech. 15:159-163, 1997. A variety of bi-specific fusion proteins can be produced using molecular engineering. In one form, the bi-specific fusion protein is monovalent, consisting of, for example, a scFv with a single binding site for one antigen and a Fab fragment with a single binding site for a second antigen. In another form, the bi-specific fusion protein is divalent, consisting of, for example, an IgG with two binding sites for one antigen and two scFv with two binding sites for a second antigen.

Functional bi-specific single-chain antibodies (bscAb), also called diabodies, can be produced in mammalian cells using recombinant methods. See, *e.g.,* Mack et al., Proc. Natl. Acad. Sci. USA 92: 7021-7025, 1995. For example, bscAb are produced by joining two single-chain Fv fragments via a glycine-serine linker using recombinant methods. The V light-chain (V_{L}) and V heavy-chain (V_{H}) domains of two antibodies of interest are isolated using standard PCR methods. The V_{L} and V_{H} cDNA's obtained from each hybridoma are then joined to form a single-chain fragment in a two-step fusion PCR. The first PCR step introduces the (Gly₄-Ser₁)₃ linker, and the second step joins the V_{L} and V_{H} amplicons. Each single chain molecule is then cloned into a bacterial expression vector. Following amplification, one of the single-chain molecules is excised and sub-cloned into the other vector, containing the second single-chain molecule of interest. The resulting bscAb fragment is subcloned into an eukaryotic expression vector. Functional protein expression can be obtained by transfecting the vector into Chinese hamster ovary cells. Bi-specific fusion proteins are prepared in a similar manner. Bi-specific single-chain antibodies and bi-specific fusion proteins are included within the scope of the present invention.

Bi-specific fusion proteins linking two or more different single-chain antibodies or antibody fragments are produced in similar manner.

Large quantities of bscAb and fusion proteins can be produced using Escherichia coli expression systems. See, *e.g.,* Zhenping et al., Biotechnology, 14: 192-196, 1996. A functional bscAb can be produced by the coexpression in E. coli of two "cross-over" scFv fragments in which the V_{L} and V_{H} domains for the two fragments are present on different polypeptide chains. The V light-chain (V_{L}) and V heavy-chain (V_{H}) domains of two antibodies of interest are isolated using standard PCR methods. The cDNA's are then ligated into a bacterial expression vector such that C-terminus of the V_{L} domain of the first antibody of interest is ligated via a linker to the N-terminus of the V_{H} domain of the second antibody. Similarly, the C-terminus of the V_{L} domain of the second antibody of interest is ligated via a linker to the N-terminus of the V_{H} domain of the first antibody. The resulting dicistronic operon is placed under transcriptional control of a strong promoter, *e.g*., the E. coli alkaline phosphatase promoter which is inducible by phosphate starvation. Alternatively, single-chain fusion constructs have successfully been expressed in *E. coli* using the lac promoter and a medium consisting of 2% glycine and 1% Triton X-100. See, *e.g.,* Yang et al., Appl. Environ. Microbiol. 64: 2869-2874, 1998. An *E. coli,* heat-stable, enterotoxin II signal sequence is used to direct the peptides to the periplasmic space. After secretion, the two peptide chains associate to form a non-covalent heterodimer which possesses both antigen binding specificities. The bscAb is purified using standard procedures known in the art, *e.g*., Staphylococcal protein A chromatography.

Functional bscAb and fusion proteins also can be produced in the milk of transgenic livestock. See, *e.g.,* Colman, A., Biochem. Soc. Symp. 63: 141-147, 1998; U.S. Pat. No. 5,827,690. The bscAb fragment, obtained as described above, is cloned into an expression vector containing a promoter sequence that is preferentially expressed in mammary epithelial cells. Examples include, but are not limited to, promoters from rabbit, cow and sheep casein genes, the cow α-lactoglobulin gene, the sheep β-lactoglobulin gene and the mouse whey acid protein gene. Preferably, the inserted bscAb is flanked on its 3' side by cognate genomic sequences from a mammary-specific gene. This provides a polyadenylation site and transcript-stabilizing sequences. The expression cassette is then injected into the pronuclei of fertilized, mammalian eggs, which are then implanted into the uterus of a recipient female and allowed to gestate. After birth, the progeny are screened for the presence of the introduced DNA by Southern analysis. Milk from transgenic females is analyzed for the presence and functionality of the bscAb using standard immunological methods known in the art. The bscAb can be purified from the milk using standard methods known in the art. Transgenic production of bscAb in milk provides an efficient method for obtaining large quantities of bscAb.

Functional bscAb and fusion proteins also can be produced in transgenic plants. See, *e.g.,* Fiedler et al., Biotech., 13: 1090-1093, 1995; Fiedler et al., Immunotechnology, 3: 205-216, 1997. Such production offers several advantages including low cost, large scale output and stable, long term storage. The bscAb fragment, obtained as described above, is cloned into an expression vector containing a promoter sequence and encoding a signal peptide sequence, to direct the protein to the endoplasmic reticulum. A variety of promoters can be utilized, allowing the practitioner to direct the expression product to particular locations within the plant. For example, ubiquitous expression in tobacco plants can be achieved by using the strong cauliflower mosaic virus 35S promoter, while organ specific expression is achieved via the seed specific legumin B4 promoter. The expression cassette is transformed according to standard methods known in the art. Transformation is verified by Southern analysis. Transgenic plants are analyzed for the presence and functionality of the bscAb using standard immunological methods known in the art. The bscAb can be purified from the plant tissues using standard methods known in the art.

Additionally, transgenic plants facilitate long term storage of bscAb and fusion proteins. Functionally active scFv proteins have been extracted from tobacco leaves after a week of storage at room temperature. Similarly, transgenic tobacco seeds stored for 1 year at room temperature show no loss of scFv protein or its antigen binding activity.

Functional bscAb and fusion proteins also can be produced in insect cells. See, *e.g.,* Mahiouz et al., J. Immunol. Methods, 212: 149-160,1998. Insect-based expression systems provide a means of producing large quantities of homogenous and properly folded bscAb. The baculovirus is a widely used expression vector for insect cells and has been successfully applied to recombinant antibody molecules. See, *e.g.,* Miller, L. K., Ann. Rev. Microbiol., 42: 177, 1988; Bei et al., J. Immunol. Methods, 186: 245, 1995. Alternatively, an inducible expression system can be utilized by generating a stable insect cell line containing the bscAb construct under the transcriptional control of an inducible promoter. See, *e*.*g*., Mahiouz et al., J. Immunol. Methods, 212: 149-160, 1998. The bscAb fragment, obtained as described above, is cloned into an expression vector containing the Drosphila metallothionein promoter and the human HLA-A2 leader sequence. The construct is then transfected into D. melanogaster SC-2 cells. Expression is induced by exposing the cells to elevated amounts of copper, zinc or cadmium. The presence and functionality of the bscAb is determined using standard immunological methods known in the art. Purified bscAb is obtained using standard methods known in the art.

The structure of BsAbs may be further altered to improve pharmacokinetics, effector functions, and binding affinity or avidity.

The present disclosure further provides compositions and methods for treating a disorder selected from the group consisting of a carcinoma, a sarcoma, a glioma, a lymphoma, a leukemia, or a skin cancer. The carcinoma can be selected from the group consisting of a skin, an esophageal, a gastric, a colonic, a rectal, a pancreatic, a lung, a breast, an ovarian, a urinary bladder, an endometrial, a cervical, a testicular, a renal, an adrenal or a liver cancer. The B-cell related disease may be an indolent form of B-cell lymphoma, an aggressive form of B-cell lymphoma, non-Hodgkin's lymphoma, a chronic lymphocytic leukemia, an acute lymphocytic leukemia, a Waldenström's macroglobulinemia, or a multiple myeloma. In addition, the B-cell related disease can be a human or a veterinary type of disease. On the other hand, the T cell related disease may be a human or veterinary T-cell leukemia, skin psoriasis, psoriatic arthritis or mycosis fungoides. The metabolic disease can be an amyloidosis. The neurodegenerative disease can be an Alzheimer's disease.

A tumor-associated antigen can be associated with any type of disease as mentioned above and may be selected from the group consisting of CD2, CD3, CD8, CD10, CD19, CD20, CD21, CD22, CD23, CD24, CD25, CD30, CD33, CD37, CD38, CD40, CD45Ro, CD48, CD52, CD55, CD59, CD70, CD74, CD80, CD86, CD138, CD147, HLA-DR, CEA, CSAp, CA-125, TAG-72, EFGR, HER2, HER3, HER4, IGF-1R, c-Met, PDGFR, MUC1, MUC2, MUC3, MUC4, TNFR1, TNFR2, NGFR, Fas (CD95), DR3, DR4, DR5, DR6, VEGF, PIGF, tenascin, ED-B fibronectin, PSMA, PSA, carbonic anhydrase IX, and IL-6.

Tumor-associated markers have been categorized by Herberman (see, *e.g.,* Immunodiagnosis of Cancer, in THE CLINICAL BIOCHEMISTRY OF CANCER, Fleisher (ed.), American Association of Clinical Chemists, 1979) in a number of categories including oncofetal antigens, placental antigens, oncogenic or tumor virus associated antigens, tissue associated antigens, organ associated antigens, ectopic hormones and normal antigens or variants thereof. Occasionally, a sub-unit of a tumor-associated marker is advantageously used to raise antibodies having higher tumor-specificity, *e.g.,* the beta-subunit of human chorionic gonadotropin (HCG) or the gamma region of carcinoembryonic antigen (CEA), which stimulate the production of antibodies having a greatly reduced cross-reactivity to non-tumor substances as disclosed in U.S. Patent Nos. 4,361,644 and 4,444,744. Markers of tumor vasculature (e.g., VEGF, P1GF, and ED-B fibronectin), of tumor necrosis, of membrane receptors (*e.g*., folate receptor, EGFR), of transmembrane antigens (*e.g*., PSMA), and of oncogene products can also serve as suitable tumor-associated targets for antibodies or antibody fragments. Markers of normal cell constituents which are overexpressed on tumor cells, such as B-cell complex antigens, as well as cytokines expressed by certain tumor cells (*e.g.,* IL-2 receptor in T-cell malignancies and IL-6 expressed by certain tumor cells and also involved in cachexia related, it has been proposed, to an inflammatory process) are also suitable targets for the antibodies and antibody fragments of this invention. See, for example, Trikha et al., Clin Cancer Res. ;9:4653-65 (2003).

Also of use are antibodies against markers or products of oncogenes, or antibodies against angiogenesis factors, such as VEGF, P1GF, and ED-B fibronectin. VEGF antibodies are described in U.S. Patent Nos. 6,342,221; 5,965,132; and 6,004,554. ED-B fibronectin antibodies, initially referred by Mariani et al. (Cancer 80: 2378-84, 1997) as an oncofetal fibronectin, are disclosed in Santimaria, M. et al., Clin. Cancer Res. 9(2): 571-579,2003; WO 97/45544A1; WO 03/055917A2; WO 01/83816A2; WO 01/62298A2; WO 99/58570A2; WO 01/62800A1; and U.S. patent publication No. 20030045681A1. Antibodies against certain immune response modulators, such as antibodies to CD40, are described in Todryk et al., J. Immunol. Meth. 248:139-147, 2001; and Turner et al., J. Immunol. 166:89-94,2001. Other antibodies suitable for combination therapy include anti-necrosis antibodies as described in Epstein *et al.,* see *e.g.,* U.S. Patent Nos. 5,019,368; 5,882,626; and 6,017,514. An example of a T cell marker for arthritic psoriasis is CD45Ro and is described by Veale, D.J. et al. in Ann. Rheum. Dis. 53(7): 450-454,1994.

The present disclosure further provides compositions and methods for treating an autoimmune disease or other immune disorder, such as an acute or chronic inflammation (such as Crohn's Disease, ulcerative colitis, psoriasis, chronic bronchitis, asthma, emphysema, myositis, or polymyositis) or an immune dysregulation disease (graft versus host disease, organ transplant rejection, cachexia, septicemia, atherosclerosis, etc.). Immunotherapy of autoimmune disorders using antibodies which target B-cells is described in PCT Application Publication No. WO 00/74718, which claims priority to U.S. Provisional Application Serial No. 60/138,284. Exemplary autoimmune diseases are acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcalnephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitisubiterans, Sjogren's syndrome, primary biliary cirrhosis, Hashimoto's thyroiditis, thyrotoxicosis, scleroderma, chronic active hepatitis, polymyositis/dermatomyositis, polychondritis, parnphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis, and fibrosing alveolitis.

BsAbs that can crosslink any two of these cell surface markers are considered. In addition, BsAbs involving VEGF and other angiogenesis-inhibiting antibodies, both in various combinations of themselves or with other inhibiting antibodies (*e.g*., P1GF and ED-B fibronectin), are included. It is noted that some of these cell surface markers are not restricted to lymphomas, but are more common to solid tumors. For example, overexpression of HER2 occurs in various human cancers, including breast, ovary, prostate, gastric, lung, bladder, and kidney carcinomas, and CD24 has also been observed to associate with non-small cell lung cancer (Kristiansen, et al., Br. J. Cancer, 88: 231-236, 2003) as well as ovarian cancer (Kristiansen, et al., Am. J. Pathol. 161: 1215-1221, 2002). It is well-known that CA-125 is also used as a marker for ovarian cancer (see Niloff et al,. Obstet Gynecol. 64:703-7 (1984)), and it has now been found that CSAp is likewise related to CA-125 and can be used as a target molecule for ovarian as well as other cancers, particularly colorectal cancer. See Modrak et al. "Identification of a Mu-9 (Anti-Colon-specific Antigen-p)-Reactive Peptide Having Homology to CA125 (MUC16), submitted to Cancer Research (1994).

Examples of therapeutic agents include antibodies, antibody fragments, drugs, including chemotherapeutic agents, toxins, enzymes, enzyme-inhibitors, nucleases, hormones, hormone antagonists, immunomodulators, cytokines, chelators, boron compounds, uranium atoms, photoactive agents and radionuclides.

Useful diagnostic/detection agents include, but are not limited to, radioisotopes, dyes (such as with the biotin-streptavidin complex), radiopaque materials (e.g., iodine, barium, gallium, and thallium compounds and the like), contrast agents, fluorescent compounds or molecules and enhancing agents (e.g., paramagnetic ions) for magnetic resonance imaging (MRI). U.S. Patent No. 6,331,175 describes MRI technique and the preparation of antibodies conjugated to a MRI enhancing agent. Preferably, the diagnostic/detection agents are selected from the group consisting of radioisotopes for nuclear imaging, intraoperative and endoscopic detection; enhancing agents for use in magnetic resonance imaging or in ultrasonography; radiopaque and contrast agents for X-rays and computed tomography; and fluorescent compounds for fluoroscopy, including endoscopic fluoroscopy.

Chemotherapeutic agents, for the purpose of this disclosure, include all known chemotherapeutic agents. Known chemotherapeutic agents include, at least, the taxanes, nitrogen mustards, ethylenimine derivatives, alkyl sulfonates, nitrosoureas, triazenes; folic acid analogs, pyrimidine analogs, purine analogs, antisense oligonucleotides, antagonists or inhibitors of transcription factors, alkaloids, antibiotics, enzymes, platinum coordination complexes, COX-2 inhibitors, apoptotic agents, substituted urea, methyl hydrazine derivatives, adrenocortical suppressants, or antagonists. More specifically, the chemotherapeutic agents may be steroids, progestins, estrogens, antiestrogens, or androgens. Even more specifically, the chemotherapy agents may be actinomycin, azaribine, anastrozole, azacytidine, bleomycin, bryostatin-1, busulfan, carmustine, celebrex, chlorambucil, cisplatin, irinotecan (CPT-11), carboplatin, cladribine, cyclophosphamide, cytarabine, dacarbazine, docetaxel, dacarbazine, dactinomycin, daunorubicin, dexamethasone, diethylstilbestrol, doxorubicin, ethinyl estradiol, estramustine, etoposide, floxuridine, fludarabine, flutamide, fluorouracil, fluoxymesterone, gemcitabine, hydroxyprogesterone caproate, hydroxyurea, idarubicin, ifosfamide, L-asparaginase, leucovorin, lomustine, mechlorethamine, medroprogesterone acetate, megestrol acetate, melphalan, mercaptopurine, methotrexate, mitoxantrone, mithramycin, mitomycin, mitotane, oxaliplatin, phenyl butyrate, prednisone, procarbazine, paclitaxel, pentostatin, semustine streptozocin, SN-38, tamoxifen, taxanes, taxol, testosterone propionate, thalidomide, thioguanine, thiotepa, teniposide, topotecan, uracil mustard, vinblastine, vinorelbine or vincristine.

Suitable chemotherapeutic agents are described in REMINGTON'S PHARMACEUTICAL SCIENCES, 19th Ed. (Mack Publishing Co. 1995), and in GOODMAN AND GILMAN'S THE PHARMACOLOGICAL BASIS OF THERAPEUTICS, 7th Ed. (MacMillan Publishing Co. 1985), as well as revised editions of these publications. Other suitable chemotherapeutic agents, such as experimental drugs, are known to those of skill in the art.

The toxin may be ricin, abrin, ribonuclease, DNase I, Staphylococcal enterotoxin-A, pokeweed antiviral protein, gelonin, diphtherin toxin, Pseudomonas exotoxin, or Pseudomonas endotoxin.

Enzymes are also useful therapeutic agents and are selected from the group consisting of malate dehydrogenase, staphylococcal nuclease, delta-V-steroid isomerase, yeast alcohol dehydrogenase, α-glycerophosphate dehydrogenase, triose phosphate isomerase, horseradish peroxidase, alkaline phosphatase, asparaginase, glucose oxidase, β-galactosidase, ribonuclease, urease, catalase, glucose-6-phosphate dehydrogenase, glucoamylase and acetylcholinesterase.

As used herein, the term "immunomodulator" includes cytokines, stem cell growth factors, lymphotoxins, such as tumor necrosis factor (TNF), and hematopoietic factors, such as interleukins (e.g., interleukin-1 (IL-1), IL-2, IL-3, IL-6, IL-10, IL-12, IL-18 and IL-21), colony stimulating factors (*e.g*., granulocyte-colony stimulating factor (G-CSF) and granulocyte macrophage-colony stimulating factor (GM-CSF)), interferons (e.g., interferons-α, -β and -γ), the stem cell growth factor designated "S1 factor", and erythropoietin and thrombopoietin. Examples of suitable immunomodulator moieties include IL-2, IL-6, IL-10, IL-12, IL-18, IL-21, interferon-y, TNF-α, and the like. Alternatively, subjects can receive invention compositions and a separately administered cytokine, which can be administered before, concurrently or after administration of the invention compositions. The invention compositions may also be conjugated to the immunomodulator.

A cytokine, for the purposes of this disclosure, include all known cytokines including, at least, IL-1, IL-2, IL-3, IL-6, IL-10, IL-12, IL-18, IL-21, interferon-α, interferon-β, and interferon-γ. It may also be a colony stimulating factor, such as GM-CSF, G-CSF, erythropoietin, thrombopoietin, and the like.

Additionally, a chelator such as DTPA, DOTA, TETA, or NOTA or a suitable peptide, to which a detectable label, such as a fluorescent molecule, or cytotoxic agent, such as a heavy metal or radionuclide, can be conjugated. For example, a therapeutically useful immunoconjugate can be obtained by conjugating a photoactive agent or dye to an antibody composite. Fluorescent compositions, such as fluorochrome, and other chromogens, or dyes, such as porphyrins sensitive to visible light, have been used to detect and to treat lesions by directing the suitable light to the lesion. In therapy, this has been termed photoradiation, phototherapy, or photodynamic therapy (Jori et al. (eds.), PHOTODYNAMIC THERAPY OF TUMORS AND OTHER DISEASES (Libreria Progetto, 1985); van den Bergh, Chem. Britain 22:430, 1986). Moreover, monoclonal antibodies have been coupled with photoactivated dyes for achieving phototherapy. Mew et al., J. Immunol. 130: 1473, 1983*; idem.,* Cancer Res. 45: 4380, 1985; Oseroff et al., Proc. Natl. Acad. Sci. USA 83: 8744,1986; *idem.,* Photochem. Photobiol. 46: 83,1987; Hasan et al., Prog. Clin. Biol. Reps. 288: 471, 1989; Tatsuta et al., Lasers Surg. Med. 9: 422, 1989; Pelegrin et al., Cancer 67: 2529,1991. However, these earlier studies did not include use of endoscopic therapy applications, especially with the use of antibody fragments or subfragments. Thus, contemplated herein is the therapeutic use of immunoconjugates comprising photoactive agents or dyes. Thus, the present therapeutic methods may include the therapeutic use of immunoconjugates comprising photoactive agents or dyes. Endoscopic methods of detection and therapy are described in U.S. patent Nos. 4,932,412; 5,525,338; 5,716,595; 5,736,119; 5,922,302; 6,096,289; and 6,387,350.

Any useful nuclide may be used within the scope of the invention. Particularly preferred are radionuclides that have useful diagnostic or therapeutic properties, such as indium-111 or yttrium-90, respectively. Other useful nuclides include, but are not limited to, F-18, P-32, Sc-47, Cu-62, Cu-64, Cu-67, Ga-67, Ga-68, Y-86, Y-90, Zr-89, Tc-99m, Pd-109, Ag-111, In-111, I-123, I-125, I-131, Sm-153, Gd-155, Gd-157, Tb-161, Lu-177, Re-186, Re-188, Pt-197, Pb-212, Bi-212, Bi-213, Ra-223, Ac-225, As-72, As-77, At-211, Au-198, Au-199, Bi-212, Br-75, Br-76B, C-11, Co-55Co, Dy-166, Er-169, F-18, Fe-52, Fe-59, Ga-67, Ga-68, Gd -154-158, Ho-166, I-120, I-121, I-124, In-110, In-111, Iri194, Lu-177, Mn-51, Mn-52, Mo-99, N-13, O-15, P-32, P-33, Pb-211, Pb-212, Pd-109, Pm-149, Pr-142, Pr-143, Rb-82, Re-189, Rh-105, Sc-47, Se-75, Sr-83, Sr-89, Tb-161, Tc-94, Tc-99, Y-86, Y-90 or Zr-89.

For example, suitable diagnostic radionuclides include In-110, In-111, Lu-177, F-18, Fe-52, Cu-62, Cu-64, Cu-67, Ga-67, Ga-68, Y-86, Y-90, Zr-89, Tc-94m, Tc-94, Tc-99m, I-120, I-123, I-124, I-125, I-131, Gd-154-158, P-32, C-11, N-13, O-15, Re-186, Re-188, Mn-51, Mn-52m, Co-55, As-72, Br-75, Br-76, Rb-82m, Zr-89 and Sr-83. A typical diagnostic radionuclide emits particles and/or positrons having between 25-10,000 keV.

Additionally, suitable therapeutic radionuclides include, but are not limited to In-111, Lu-177, Bi-212, Bi-213, At-211, Cu-62, Cu-64, Cu-67, Y-90, I-125, I-131, P-32, P-33, Sc-47, Ag, 67Ga-111, Pr-142, Sm-153, Tb-161, Dy-166, Ho-166, Re-186, Re-188, Re-189, Pb-212, Ra-223, Ac-225, Fe-59, Se-75, As-77, Sr-89, Mo-99, Rh-105, Pd-109, Pr-143, Pm-149, Er-169, Ir-194, Au-198, Au-199, Ac-225 and Pb-211. A typical therapeutic cation emits particles and/or positrons having between 20-10,000 keV.

Maximum decay energies of useful beta-particle-emitting nuclides are preferably 20-5,000 keV, more preferably 100-4,000 keV, and most preferably 500-2,500 keV. Also preferred are radionuclides that substantially decay with Auger-emitting particles. For example, Co-58, Ga-67, Br-80m, Tc-99m, Rh-103m, Pt-109, In-111, Sb-119, I-125, Ho-161, Os-189m and Ir-192. Decay energies of useful Auger-particle-emitting nuclides are preferably < 1,000 keV, more preferably < 100 keV, and most preferably < 70 keV. Also preferred are radionuclides that substantially decay with generation of alpha-particles. Such radionuclides include, but are not limited to: Dy-152, At-211, Bi-212, Ra-223, Rn-219, Po-215, Bi-211, Ac-225, Fr-221, At-217, Bi-213 and Fm-255. Decay energies of useful alpha-particle-emitting radionuclides are preferably 2,000-10,000 keV, more preferably 3,000-8,000 keV, and most preferably 4,000-7,000 keV.

Other useful therapeutic agents include metals, such as those as part of a photodynamic therapy, and nuclides, such as those valuable in therapies based on neutron capture procedures. Specifically, zinc, aluminum, gallium, lutetium and palladium are useful for photodynamic therapy and B-10, Gd-157 and U-235 are useful for neutron capture therapy.

Metals are also useful in diagnostic agents, including those for magnetic resonance imaging techniques. These metals include, but are not limited to: Gadolinium, manganese, iron, chromium, copper, cobalt, nickel, dysprosium, rhenium, europium, terbium, holmium and neodymium. In order to load an antibody component with radioactive metals or paramagnetic ions, it may be necessary to react it with a reagent having a long tail to which are attached a multiplicity of chelating groups for binding the ions. Such a tail can be a polymer such as a polylysine, polysaccharide, or other derivatized or derivatizable chain having pendant groups to which can be bound chelating groups such as, *e.g.*, ethylenediaminetetraacetic acid (EDTA), diethylenetriaminepentaacetic acid (DTPA), porphyrins, polyamines, crown ethers, bis-thiosemicarbazones, polyoximes, and like groups known to be useful for this purpose. Chelates are coupled to the peptide antigens using standard chemistries. The chelate is normally linked to the antibody by a group which enables formation of a bond to the molecule with minimal loss of immunoreactivity and minimal aggregation and/or internal cross-linking. Other, more unusual, methods and reagents for conjugating chelates to antibodies are disclosed in U.S. Patent 4,824,659 to Hawthorne, entitled "Antibody Conjugates," issued April 25,1989. Particularly useful metal-chelate combinations include 2-benzyl-DTPA and its monomethyl and cyclohexyl analogs, used with diagnostic isotopes in the general energy range of 20 to 2,000 keV. The same chelates, when complexed with non-radioactive metals, such as manganese, iron and gadolinium are useful for MRI, when used along with the antibodies of the invention. Macrocyclic chelates such as NOTA, DOTA, and TETA are of use with a variety of metals and radiometals, most particularly with radionuclides of gallium, yttrium and copper, respectively. Such metal-chelate complexes can be made very stable by tailoring the ring size to the metal of interest. Other ring-type chelates such as macrocyclic polyethers, which are of interest for stably binding nuclides, such as ²²³Ra for RAIT are encompassed by the invention.

Therapeutically useful immunoconjugates can be obtained by conjugating photoactive agents or dyes to an antibody composite. Fluorescent and other chromogens, or dyes, such as porphyrins sensitive to visible light, have been used to detect and to treat lesions by directing the suitable light to the lesion. In therapy, this has been termed photoradiation, phototherapy, or photodynamic therapy (Jori et al., eds., Photodynamic Therapy of Tumors and Other Diseases (Libreria Progetto 1985); van den Bergh, Chem. Britain 22: 430,1986). Moreover, monoclonal antibodies have been coupled with photoactivated dyes for achieving phototherapy. Mew et al., J. Immunol. 130: 1473, 1983; idem., Cancer Res. 45:4380, 1985; Oseroff et al., Proc. Natl. Acad. Sci. USA 83: 8744, 1986; idem., Photochem. Photobiol. 46:83, 1987; Hasan et al., Prog. Clin. Biol. Res. 288: 471, 1989; Tatsuta et al., Lasers Surg. Med. 9: 422, 1989; Pelegrin et al., Cancer 67: 2529, 1991. However, these earlier studies did not include use of endoscopic therapy applications, especially with the use of antibody fragments or subfragments. Thus, the present invention contemplates the therapeutic use of immunoconjugates comprising photoactive agents or dyes.

Paramagnetic ions suitable for the present invention include include chromium (III), manganese (II), iron (III), iron (II, cobalt (II), nickel (II), copper (II), neodymium (III), samarium (III), ytterbium (III), gadolinium (III), vanadium (II), terbium (III), dysprosium (III), holmium (III) and erbium (III), with gadolinium being particularly preferred.

Ions useful in other contexts, such as X-ray imaging, include but are not limited to lanthanum (III), gold (III), lead (II), and especially bismuth (III). Fluorescent labels include rhodamine, fluorescein and renographin. Rhodamine and fluorescein are often linked via an isothiocyanate intermediate.

Radiopaque and contrast materials are used for enhancing X-rays and computed tomography, and include iodine compounds, barium compounds, gallium compounds, thallium compounds, etc. Specific compounds include barium, diatrizoate, ethiodized oil, gallium citrate, iocarmic acid, iocetamic acid, iodamide, iodipamide, iodoxamic acid, iogulamide, iohexol, iopamidol, iopanoic acid, ioprocemic acid, iosefamic acid, ioseric acid, iosulamide meglumine, iosemetic acid, iotasul, iotetric acid, iothalamic acid, iotroxic acid, ioxaglic acid, ioxotrizoic acid, ipodate, meglumine, metrizamide, metrizoate, propyliodone, and thallous chloride.

A "pharmaceutical composition" refers to a composition comprising a drug wherein the carrier is a pharmaceutically acceptable carrier, while a "veterinary composition" is one wherein the carrier is a veterinarily acceptable carrier. The term "pharmaceutically acceptable carrier" or "veterinarily acceptable carrier" includes any medium or material that is not biologically or otherwise undesirable, i.e, the carrier may be administered to an organism along with a composition or compound of the invention without causing any undesirable biological effects or interacting in a deleterious manner with the complex or any of its components or the organism. Examples of pharmaceutically acceptable reagents are provided in The United States Pharmacopeia, The National Formulary, United States Pharmacopeial Convention, Inc., Rockville, MD 1990 and in Pharmaceutical Dosage Forms & Drug Delivery Systems, 7th Edition, Ansel et al., editors, Lippincott Williams & Wilkins, 1999.

The drug (*i.e.*, targetable construct or complex) is included in the pharmaceutical composition in an amount sufficient to produce the desired effect upon the patient. The pharmaceutical compositions of the invention can further comprise other chemical components, such as diluents and excipients. A "diluent" is a chemical compound diluted in a solvent, preferably an aqueous solvent, that facilitates dissolution of the drug in the solvent, and it may also serve to stabilize the biologically active form of the drug or one or more of its components. Salts dissolved in buffered solutions are utilized as diluents in the art. For example, preferred diluents are buffered solutions containing one or more different salts. A preferred buffered solution is phosphate buffered saline (particularly in conjunction with compositions intended for pharmaceutical administration), as it mimics the salt conditions of human blood. Since buffer salts can control the pH of a solution at low concentrations, a buffered diluent rarely modifies the biological activity of a biologically active peptide.

An "excipient" is any more or less inert substance that can be added to a composition in order to confer a suitable property, for example, a suitable consistency or to form a drug. Suitable excipients and carriers include, in particular, fillers such as sugars, including lactose, sucrose, mannitol, or sorbitol cellulose preparations such as, for example, maize starch, wheat starch, rice starch, agar, pectin, xanthan gum, guar gum, locust bean gum, hyaluronic acid, casein potato starch, gelatin, gum tragacanth, polyacrylate, methyl cellulose, hydroxypropylmethyl-cellulose, sodium carboxymethylcellulose, and/or polyvinylpyrrolidone (PVP). If desired, disintegrating agents can also be included, such as cross-linked polyvinylpyrrolidone, agar, or alginic acid or a salt thereof such as sodium alginate. Other suitable excipients and carriers include hydrogels, gellable hydrocolloids, and chitosan. Chitosan microspheres and microcapsules can be used as carriers. See WO 98/52547 (which describes microsphere formulations for targeting compounds to the stomach, the formulations comprising an inner core (optionally including a gelled hydrocolloid) containing one or more active ingredients, a membrane comprised of a water insoluble polymer (*e.g.*, ethylcellulose) to control the release rate of the active ingredient(s), and an outer layer comprised of a bioadhesive cationic polymer, for example, a cationic polysaccharide, a cationic protein, and/or a synthetic cationic polymer; U.S. Patent No. 4,895,724. Typically, chitosan is cross-linked using a suitable agent, for example, glutaraldehyde, glyoxal, epichlorohydrin, and succinaldehyde. Compositions employing chitosan as a carrier can be formulated into a variety of dosage forms, including pills, tablets, microparticles, and microspheres, including those providing for controlled release of the active ingredient(s). Other suitable bioadhesive cationic polymers include acidic gelatin, polygalactosamine, polyamino acids such as polylysine, polyhistidine, polyornithine, polyquaternary compounds, prolamine, polyimine, diethylaminoethyldextran (DEAE), DEAE-imine, DEAE-methacrylate, DEAE-acrylamide, DEAE-dextran, DEAE-cellulose, poly-p-aminostyrene, polyoxethane, copolymethacrylates, polyamidoamines, cationic starches, polythiodiethylaminomethylethylene and polyvinylpyridine.

The targetable constructs and complexes of the invention can be formulated in any suitable manner. The targetable constructs and complexes may be uniformly (homogeneously) or non-uniformly (heterogenously) dispersed in the carrier. Suitable formulations include dry and liquid formulations. Dry formulations include freeze dried and lyophilized powders, which are particularly well suited for aerosol delivery to the sinuses or lung, or for long term storage followed by reconstitution in a suitable diluent prior to administration. Other preferred dry formulations include those wherein a pharmaceutical composition according to the invention is compressed into tablet or pill form suitable for oral administration or compounded into a sustained release formulation. When the pharmaceutical composition is intended for oral administration but the targetable construct or complex is to be delivered to epithelium in the intestines, it is preferred that the formulation be encapsulated with an enteric coating to protect the formulation and prevent premature release of the targetable constructs and complexes included therein. As those in the art will appreciate, the pharmaceutical compositions of the invention can be placed into any suitable dosage form. Pills and tablets represent some of such dosage forms. The pharmaceutical compositions can also be encapsulated into any suitable capsule or other coating material, for example, by compression, dipping, pan coating, spray drying, *etc.* Suitable capsules include those made from gelatin and starch. In turn, such capsules can be coated with one or more additional materials, for example, and enteric coating, if desired. Liquid formulations include aqueous formulations, gels, and emulsions.

The pharmaceutical compositions of the disclosure facilitate administration of monoclonal antibodies to an organism, preferably an animal, preferably a mammal, bird, fish, insect, or arachnid. Preferred mammals include bovine, canine, equine, feline, ovine, and porcine animals, and non-human primates. Humans are particularly preferred. Multiple techniques of administering or delivering a compound exist in the art including, but not limited to, oral, rectal (*e.g.*, an enema or suppository) aerosol (*e.g.,* for nasal or pulmonary delivery), parenteral (*e.g.,* i.v., i.m., s.c.), and topical administration. Preferably, sufficient quantities of the composition or compound of the invention are delivered to achieve the intended effect. The particular amount of composition or compound to be delivered will depend on many factors, including the effect to be achieved, the type of organism to which the composition is delivered, delivery route, dosage regimen, and the age, health, and sex of the organism. As such, the particular dosage of a composition or compound of the invention included in a given formulation is left to the ordinarily skilled artisan's discretion.

Those skilled in the art will appreciate that when the pharmaceutical compositions of the present disclosure are administered as agents to achieve a particular desired biological result, which may include a therapeutic or protective effect(s) (including vaccination), it may be necessary to combine the composition or compound of the invention with a suitable pharmaceutical carrier. The choice of pharmaceutical carrier and the preparation of the composition or compound as a therapeutic or protective agent will depend on the intended use and mode of administration. Suitable formulations and methods of administration of therapeutic agents include, but are not limited to, those for oral, pulmonary, nasal, buccal, ocular, dermal, rectal, or vaginal delivery.

Depending on the mode of delivery employed, the context-dependent functional entity can be delivered in a variety of pharmaceutically acceptable forms. For example, the context-dependent functional entity can be delivered in the form of a solid, solution, emulsion, dispersion, micelle, liposome, and the like, incorporated into a pill, capsule, tablet, suppository, areosol, droplet, or spray. Pills, tablets, suppositories, areosols, powders, droplets, and sprays may have complex, multilayer structures and have a large range of sizes. Aerosols, powders, droplets, and sprays may range from small (1 micron) to large (200 micron) in size.

Pharmaceutical compositions of the present disclosure can be used in the form of a solid, a lyophilized powder, a solution, an emulsion, a dispersion, a micelle, a liposome, and the like, wherein the resulting composition contains one or more of the targetable constructs or complexes of the present invention, as an active ingredient, in admixture with an organic or inorganic carrier or excipient suitable for enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used include glucose, lactose, mannose, gum acacia, gelatin, mannitol, starch paste, magnesium trisilicate, talc, corn starch, keratin, colloidal silica, potato starch, urea, medium chain length triglycerides, dextrans, and other carriers suitable for use in manufacturing preparations, in solid, semisolid, or liquid form. In addition auxiliary, stabilizing, thickening and coloring agents and perfumes may be used. Examples of a stabilizing dry agent includes triulose, preferably at concentrations of 0.1% or greater (See, *e.g.*, U.S. Patent No. 5,314,695).

Although individual needs may vary, determination of optimal ranges for effective amounts of pharmaceutical compositions is within the skill of the art. Human doses can be extrapolated from animal studies (Katocs et al., Chapter 27 In: Remington's Pharmaceutical Sciences, 18th Ed., Gennaro, ed., Mack Publishing Co., Easton, Pa., 1990). Generally, the dosage required to provide an effective amount of a pharmaceutical composition, which can be adjusted by one skilled in the art, will vary depending on the age, health, physical condition, weight, type and extent of the disease or disorder of the recipient, frequency of treatment, the nature of concurrent therapy (if any) and the nature and scope of the desired effect(s). See, for example, Nies et al., Chapter 3 In: Goodman & Gilman's The Pharmacological Basis of Therapeutics, 9th Ed., Hardman et al., eds., McGraw-Hill, New York, N.Y., 1996)

Dosing of therapeutic compositions is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. The term "patient" is intended to encompass animals (*e.g.*, cats, dogs and horses) as well as humans. Persons of ordinary skill can easily determine optimum dosages, dosing methodologies and repetition rates. Optimum dosages may vary depending on the relative potency of individual therapeutic agents, and can generally be estimated based on EC₅₀ found to be effective in *in vitro* and *in vivo* animal models.

The range of doses (the amount of targetable construct or complex administered) is broad, since in general the efficacy of a therapeutic effect for different mammals varies widely with doses typically being 20, 30 or even 40 times smaller (per unit body weight) in man than in the rat. In general, dosage is from 0.01 *µ*g to 100 mg per kg of body weight, preferably 0.01 *µ*g to 10 mg/kg of body weight, 0.01 *µ*g to 50 mg/kg of body weight, 0.01 *µ*g to 100 mg/kg of body weight, 0.01 *µ*g to 10 mg/kg of body weight, 0.01 *µ*g to 1 mg/kg of body weight, 0.01 *µ*g to 100 *µ*g/kg of body weight, 0.01 *µ*g to to 10 *µ*g/kg of body weight, 0.01 *µ*g to 1 *µ*g/kg of body weight, 0.01 *µ*g to 10 *µ*g/kg of body weight, 0.01 *µ*g to 1 *µ*g/kg of body weight, 0.01 *µ*g to 0.1 *µ*g/kg of body weight, and ranges based on the boundaries of the preceding ranges of concentrations. Thus, for example, the preceding description of dosages encompasses dosages within the range of 10 mg to 100 mg per kg of body weight, 1.0 mg to 100 mg/kg of body weight, 0.1 mg to 100 mg/kg of body weight, *etc.*

Doses may be given once or more times daily, weekly, monthly or yearly, or even once every 2 to 20 years. Persons of ordinary skill in the art can easily estimate repetition rates for dosing based on measured residence times and concentrations of the targetable construct or complex in bodily fluids or tissues. Following successful treatment, it may be desirable to have the patient undergo maintenance therapy to prevent the recurrence of the disease state, wherein the therapeutic agent is administered in maintenance doses, ranging from 0.01 µg to 100 mg per kg of body weight, once or more daily, to once every 20 years.

The specific dose is calculated according to the approximate body weight or surface area of the patient. Other factors in determining the appropriate dosage can include the disease or condition to be treated or prevented, the severity of the disease, the route of administration, and the age, sex and medical condition of the patient. Further refinement of the calculations necessary to determine the appropriate dosage for treatment is routinely made by those skilled in the art, especially in light of the dosage information and assays disclosed herein. The dosage can also be determined through the use of known assays for determining dosages used in conjunction with appropriate dose-response data.

An individual patient's dosage can be adjusted as the progress of the disease is monitored. Blood levels of the targetable construct or complex in a patient can be measured to see if the dosage needs to be adjusted to reach or maintain an effective concentration. Pharmacogenomics may be used to determine which targetable constructs and/or complexes, and dosages thereof, are most likely to be effective for a given individual (Schmitz et al., Clinica Chimica Acta 308: 43-53, 2001; Steimer et al., Clinica Chimica Acta 308: 33-41, 2001).

Administration of the heteroconjugates of the present incention should be preferably parenteral, including intravenous, intraarterial, intraperitoneal, intramuscular, subcutaneous, intrapleural, intrathecal, intracavitary, by perfusion through a catheter or by direct intralesional injection. This may be administered once or more times daily, once or more times weekly, once or more times monthly, and once or more times annually.

### EXAMPLES

### MATERIALS AND METHODS

BsAbs were prepared by linking two parental Fab' fragments with o-phenylenedimaleimide as described in Sharkey et al., Cancer Research 63: 354-363, 2003. All *in vitro* studies were performed with Daudi, a human Burkitt's lymphoma cell line expressing CD20, CD22 and CD74. Tumor cells were grown in culture media containing RPMI 1640 (GIBCO-Invitrogen, Catalog # 21870-084) supplemented with 10% Fetal Bovine serum (Hyclone, Catalog #SH 30070.03), L-Glutamine (GIBCO- Invitrogen, Catalog # 25030-149) final concentration 2 mM, Penicillin-Streptomycin (GIBCO- Invitrogen, Catalog # 15140-122) final concentration 100 U/mL of Penicillin and 100 ug/mL of Streptomycin. A mouse anti-human IgM antibody (Southern Biotechnology Associates, Inc., Cat # 9020-01) was used as a positive control for its ability to induce apoptosis upon binding to the B-cell antigen receptor. Daudi cells growing in cell culture media served as a negative control for unaltered cell growth.

Several very sensitive and complementary flow cytometric assays were used to evaluate cell growth inhibition and apoptosis on a Guava PCA system purchased from Guava Technologies, Inc. (Hayward, CA), which also makes the reagents for these assays.

The Guava ViaCount Assay was used to determines viable cell count, total cell count and % viability by differentially staining viable and non-viable cells based on their permeability to the DNA-binding dyes provided in the Guava ViaCount Reagent.

To measure apoptosis, the Guava Nexin Assay was employed. The Annexin V-PE and Nexin 7-AAD allows the differentiation of apoptotic cells (Annexin V-PE positive) into early stage (7-AAD negative) and late stage (7-AAD positive). Annexin V is a calcium-dependent phospholipid binding protein with a high affinity for phosphatidylserine (PS). Annexin V-PE binds to the PS that has migrated to the outside of the cell membrane upon induction of apoptosis. Nexin 7-AAD is used as an indicator of membrane structural integrity as it binds to nuclear material within the cell after the membrane breakdown.

The Guava MultiCaspase Assay uses a cell-permeable, non-cytotoxic fluorochrome-conjugated inhibitor of caspases called SR-VAD-FMK to measure activated caspases. Once inside the cell, SR-VAD-FMK binds covalently to multiple caspases that have been activated in apoptosis. The resulting signal is proportional to the number of active caspase enzymes that are present in the cell. Cells with significant positive SR-VAD-FMK staining are in the early- to mid-apoptotic stages. The MultiCaspase Assay also includes 7-AAD to detect late-stage apoptotic cells and dead cells.

The Guava TUNEL Assay was used to quantitate cells at mid- to late-stage apoptosis, when DNA degradation is occurring. Terminal deoxynucleotidyl transferase (TdT) is used to catalyze the incorporation of BrdU residues into the fragmenting nuclear DNA at the 3'-hydroxyl ends by nicked end labeling. Subsequently, fluorescent TRITC-conjugated anti-BrdU antibody is added to bind to the incorporated BrdU residues, thereby labeling the apoptotic cells (TRITC-positive). The non-apoptotic cells are TRITC-negative.

Internalization studies were initially assessed with a FITC-conjugated goat anti-human Fab (Sigma, Catalog # F5512), following binding of the bispecific antibody. Due to high background caused by the FITC-conjugated goat anti-human Fab, later studies were performed with the bispecific antibody directly conjugated to the fluorochrome Alexa488. Stained cells were visualized under a fluorescent microscope.

### RESULTS AND DISCUSSION

The apoptotic effect of the three BsAbs (hLL2 x hA20, hA20 x hLL1, and hLL2 x hLL1) on Daudi cells, as determined by the Guava Nexin Assay, is shown in Figure 1. Apoptosis of Daudi cells was clearly observed with hLL2 x hA20 in a dose-dependent manner at the three concentrations tested (0.1, 1.0 and 10 µg/mL). When Daudi cells were treated with hLL2xhA20 at 10 µg/mL for 24 h, about 25% of the cells were found in early apoptosis, as assessed by the annexin V binding. By comparison, hA20 x hLL1 at the same concentration was not as effective as hLL2 x hA20 to induce apoptosis in Daudi cells at all time points. Little or no apoptotic effect was observed for hLL2 x hLL1 under similar conditions.

These results indicate that a BsAb, such as hLL2xhA20 capable of simultaneously targeting CD22 and CD20 on B cells, can induce apoptosis and reduces viable population of Daudi cells in a dose-dependent manner without the need for further crosslinking.

Table 1 summarizes the results obtained by the Guava Nexin Assay for hLL2 x hA20 on Daudi cells. The % apoptotic cells at early stage induced by hLL2 x hA20 was comparable to that of αIgM control at each of the three time points. The extent of apoptosis observed for hLL2 x hA20 at 10 µg/mL was about 7 fold higher than that observed for a mixture of hLL2 F(ab')₂ and hA20 F(ab')₂ (each 5 µg/mL) at 24 h and 2 to 3 fold higher at 48 and 72 hrs.

**TABLE 1. % APOPTOTIC CELLS AT EARLY STAGE (STAINED BY ANNEXIN V ONLY) AS DETERMINED BY GUAVA NEXIN ASSAY ON DAUDI^{a}**

| **Sample** | **[Conc]** | **24 h** | **48 h** | **72 h** |
|---|---|---|---|---|
| | | | | |
| Untreated | - | 1.4 ± 0.8 (5) | 3.5 ± 2.2 (6) | 1.6 ± 0.9 (3) |
| αIgM (positive control) | 5 µg/mL | 17.7 ± 9.2 (5) | 29.0 ± 12.6 (6) | 35.0 ± 19.6 (3) |
| hLL2 x hA20 (Batch 090402) | 10 µg/mL | 18.4 ± 8.8 (5) | 27.6 ± 6.6 (6) | 21.6 ± 10.2 (3) |
| hLL2 F(ab')₂ + hA20 F(ab')₂ | 5 µg/mL | 2.5 (1) | 10.6 ± 0.8 (2) | 10.6 ± 0.6 (2) |

| | | | | |
|---|---|---|---|---|
| ^{a}The value shown in parenthesis represents the number of times the test was performed with a different sample preparation. | | | | |

The apoptotic effect observed for hLL2 x hA20 was confirmed with a second batch as shown in Table 2.

**TABLE 2. COMPARISON OF % APOPTOTIC POPULATIONS OF DAUDI AS DETERMINED BY GUAVA NEXIN ASSAY FOR TWO BATCHES OF HLL2 X HA20**

| **Sample** | **[Conc]** | **24 h** | | | **48 h** | | | **72 h** | | |
|---|---|---|---|---|---|---|---|---|---|---|
| | µg/mL | Early stage^{a} | Late stage^{b} | Debris^{c} | Early stage^{a} | Late stage^{b} | Debris^{c} | Early stage^{a} | Late stage^{b} | Debris ^{c} |
| | | | | | | | | | | |
| Untreated | - | 2.4 | 3.9 | 3.5 | 7.1 | 4.2 | 3.0 | 2.5 | 6.9 | 0.2 |
| Batch 090402 | 10 | 29.9 | 4.7 | 1.2 | 17.1 | 5.4 | 0.2 | 11.7 | 7.2 | 0.0 |
| Batch 050703 | 10 | 35.1 | 5.1 | 2.1 | 21.4 | 5.5 | 0.0 | 14.6 | 7.5 | 0.2 |
| αIgM | 5 | 19.9 | 5.9 | 4.6 | 21.7 | 9.9 | 0.8 | 13.3 | 12.4 | 0.1 |

| | | | | | | | | | | |
|---|---|---|---|---|---|---|---|---|---|---|
| ^{a}Cells stained only by Annexin V are considered to be at early stage of apoptosis. ^{b}Cells stained by both Annexin V and 7-AAD are considered to be at late stage of apoptosis. ^{c}Stained by 7-AAD only. | | | | | | | | | | |

Preliminary results from the Guava MultiCaspase Assay and the Guava TUNEL assay also demonstrated that the apoptotic effect of hLL2 x hA20 on Daudi cells was accompanied by caspase activation and DNA fragmentation. About 11% of the cell population treated with hLL2 x hA20 (10 ug/mL) was found in mid-apoptotic stage as shown by MultiCaspase Assay at 24h (vs. 1% of untreated), and 49% positive by the Guava TUNEL Assay at 24 h (vs. 9% of untreated).

The internalization studies indicated that hLL2 x hA20 upon binding to Daudi caused aggregation of the receptors on the cell surface into distinct patches or clusters, which later internalized.

## Claims

1. A heteroconjugate comprising at least a first binding arm and a second binding arm,
wherein said first binding arm comprises an anti-CD20 monoclonal antibody or an antigen-binding fragment thereof,
wherein said second binding arm comprises an anti-CD22 monoclonal antibody or an antigen-binding fragment thereof, and
wherein said heteroconjugate is capable of inducing apoptosis in a Daudi cell when said heteroconjugate is contacted with the Daudi cell for 24 hours,
for use in a method for treatment of a B-cell related lymphoma, chronic lymphocytic leukemia or acute lymphocytic leukemia.

2. Use of a heteroconjugate comprising at least a first binding arm and a second binding arm,
wherein said first binding arm comprises an anti-CD20 monoclonal antibody or an antigen-binding fragment thereof,
wherein said second binding arm comprises an anti-CD22 monoclonal antibody or an antigen-binding fragment thereof, and
wherein said heteroconjugate is capable of inducing apoptosis in a Daudi cell when said heteroconjugate is contacted with the Daudi cell for 24 hours,
for the manufacture of a medicament for the treatment of a B-cell related lymphoma, chronic lymphocytic leukemia or acute lymphocytic leukemia.

3. The heteroconjugate for use according to claim 1 and the use according to claim 2,
wherein said anti-CD20 monoclonal antibody or antigen-binding fragment thereof comprises CDRs of the light chain and heavy chain of hA20 antibody,
and
wherein said anti-CD22 antibody or antigen-binding fragment thereof comprises CDRs of the light chain and heavy chain of hLL-2 antibody.

4. The heteroconjugate for use according to claim 1 and the use according to claim 2, wherein said anti-CD20 monoclonal antibody and anti-CD22 monoclonal antibody respectively comprise framework sequences of variable regions of light and heavy chains of a human antibody.

5. The heteroconjugate for use according to claim 1 and the use according to claim 2, wherein said first and second binding arms do not have apoptotic activity when not conjugated to each other.

6. The heteroconjugate for use according to claim 1 and the use according to claim 2, wherein said heteroconjugate comprises a fusion protein comprising said first and said second binding arm.

7. The heteroconjugate for use according to claim 1 and the use according to claim 2, wherein said first and said second binding arms are conjugated via a chemical linkage.

8. The heteroconjugate for use according to claim 1 and the use according to claim 2, wherein said heteroconjugate induces apoptosis in a dose-dependent manner without cross-linking of heteroconjugates to each other.

9. The heteroconjugate for use according to claim 1 and the use according to claim 2, wherein said heteroconjugate is a multi-specific heteroconjugate that binds to more than two antigens.

10. The heteroconjugate for use according to claim 1 and the use according to claim 2, wherein said binding arms are human, murine, chimeric, primatized or humanized antibodies or antigen-binding fragments.

11. The heteroconjugate for use according to claim 1 and the use according to claim 2, wherein said heteroconjugate has a structure selected from the group consisting of IgG x Fab', IgG x sFv, F(ab')₂ x Fab', Fab' x Fab', Fab' x sFv, (sFv x sFv)₂, sFv x sFv, diabody, triabody, tetrabody, and quintabody.

12. The heteroconjugate for use according to claim 1 and the use according to claim 2, wherein said heteroconjugate is made up of a bispecific antibody or a multispecific antibody having greater than two specific binding proteins.

13. The heteroconjugate for use according to claim 12 and the use according to claim 12, wherein said bispecific antibody has a bivalent Fab' X Fab' structure.

14. The heteroconjugate for use according to claim 1 and the use according to claim 2, wherein said heteroconjugate further comprises at least one compound selected from the group consisting of a chelator, a chemotherapeutic agent, an enzyme, a hormone, an immunomodulator, an oligonucleotide, a radionuclide, a boron compound, a photoactive agent and a toxin.

15. The heteroconjugate for use according to claim 14 and the use according to claim 14, wherein said compound is a chelator selected from the group consisting of DTPA, DOTA, TETA, NOTA and a suitable peptide to which a detectable label or a cytotoxic agent can be conjugated.

16. The heteroconjugate for use according to claim 14, and the use according to claim 14, wherein said compound is a chemotherapeutic agent selected from the group consisting of anthracyclines, taxanes, nitrogen mustards, ethylenimine derivatives, alkyl sulfonates, nitrosoureas, triazenes, folic acid analogs, pyrimidine analogs, purine analogs, antisense oligonucleotides, antagonists or inhibitors of transcription factors, alkaloids, antibiotics, enzymes, platinum coordination complexes, COX-2 inhibitors, apoptotic agents, thalidomide and its derivatives, substituted urea, methyl hydrazine derivatives, and adrenocortical suppressants or antagonists.

## Patentansprüche

1. Heterokonjugat umfassend wenigstens einen ersten bindenden Arm und einen zweiten bindenden Arm,
wobei der erste bindende Arm einen monoklonalen anti-CD20 Antikörper oder ein Antigen-bindendes Fragment davon umfasst,
wobei der zweite bindende Arm einen monoklonalen anti-CD22 Antikörper oder ein Antigen-bindendes Fragment davon umfasst, und
wobei das Heterokonjugat in der Lage ist, Apoptose in einer Daudi-Zelle zu induzieren, wenn das Heterokonjugat mit der Daudi-Zelle für 24 Stunden kontaktiert wird,
zur Verwendung in einem Verfahren zur Behandlung eines B-Zellen betreffenden Lymphoms, chronischer Lymphozytenleukämie oder akuter Lymphozytenleukämie.

2. Verwendung eines Heterokonjugats umfassend wenigstens einen ersten bindenden Arm und einen zweiten bindenden Arm,
wobei der erste bindende Arm einen monoklonalen anti-CD20 Antiköprer oder ein Antigen-bindendes Fragment davon umfasst,
wobei der zweite Antigen-bindende Arm einen monoklonalen anti-CD22-Antikörper oder ein Antigen-bindendes Fragment davon umfasst, und
wobei das Heterokonjugat in der Lage ist, Apoptose in einer Daudi-Zelle zu induzieren, wenn das Heterokonjugat mit der Daudi-Zelle für 24 Stunden kontaktiert wird,
für die Herstellung eines Medikaments für die Behandlung eines B-Zellen betreffenden Lymphoms, chronischer Lymphozytenleukämie oder akuter Lymphozytenleukämie.

3. Heterokonjugat zur Verwendung nach Anspruch 1 und die Verwendung nach Anspruch 2,
wobei der monoklonale anti-CD20 Antikörper und das Antigen-bindende Fragment davon CDRs der leichten Kette und der schweren Kette von hA20-Antikörper umfassen,
und
wobei der anti-CD22 Antikörper oder das Antigen-bindende Fragment davon CDRs der leichten Kette und der schweren Kette von hLL-2 Antikörper umfassen.

4. Heterokonjugat zur Verwendung nach Anspruch 1 und die Verwendung nach Anspruch 2, wobei der monoklonale anti-CD20 Antikörper und der monoklonale anti-CD22 Antikörper jeweils Gerüstsequenzen von variablen Regionen von leichten und schweren Ketten eines menschlichen Antikörpers umfassen.

5. Heterokonjugat zur Verwendung nach Anspruch 1 und Verwendung nach Anspruch 2, wobei der erste und der zweite bindende Arm keine apoptotische Aktivität aufweisen, wenn sie nicht miteinander konjugiert sind.

6. Heterokonjugat zur Verwendung nach Anspruch 1 und Verwendung nach Anspruch 2, wobei das Heterokonjugat ein Fusionsprotein umfasst, das den ersten und den zweiten bindenden Arm umfasst.

7. Heterokonjugat zur Verwendung nach Anspruch 1 und Verwendung nach Anspruch 2, wobei der erste und der zweite bindende Arm vermittels einer chemischen Bindung konjugiert sind.

8. Heterokonjugat zur Verwendung nach Anspruch 1 und Verwendung nach Anspruch 2, wobei das Heterokonjugat Apoptose in einer dosisabhängigen Art und Weise ohne Quervernetzung von Heterokonjugaten miteinander induziert.

9. Heterokonjugat zur Verwendung nach Anspruch 1 und Verwendung nach Anspruch 2, wobei das Heterokonjugat ein multi-spezifisches Heterokonjugat ist, das an mehr als zwei Antigene bindet.

10. Heterokonjugat zur Verwendung nach Anspruch 1 und Verwendung nach Anspruch 2, wobei die bindenden Arme menschliche, murine, chimäre, primatisierte oder humanisierte Antikörper oder Antigen-Fragmente sind.

11. Heterokonjugat zur Verwendung nach Anspruch 1 und Verwendung nach Anspruch 2, wobei das Heterokonjugat eine Struktur aufweist, die ausgewählt ist aus der Gruppe bestehend aus IgG x Fab', IgG x sFv, F(ab')₂ x Fab', Fab' x Fab', Fab' x sFv, (sFv x sFv)₂, sFv x sFv, Diabody, Triabody, Tetrabody und Quintabody.

12. Heterokonjugat zur Verwendung nach Anspruch 1 und Verwendung nach Anspruch 2, wobei das Heterokonjugat aus einem bispezifischen Antikörper oder einem multispezfisichen Antikörper mit mehr als zwei spezifischen Bindungsproteinen hergestellt ist.

13. Heterokonjugat zur Verwendung nach Anspruch 12 und Verwendung nach Anspruch 12, wobei der bispezifische Antikörper eine bivalente Fab' X Fab'-Struktur aufweist.

14. Heterokonjugat zur Verwendung nach Anspruch 1 und Verwendung nach Anspruch 2, wobei das Heterokonjugat weiter wenigstens eine Verbindung umfasst, die ausgewählt ist aus der Gruppe aus einem Chelator, aus einem chemotherapeutischen Agens, einem Enzym, einem Hormon, einem Immunmodulator, einem Oligonukleotid, einem Radionuklid, einer Bor-Verbindung, einem photoaktiven Agens und einem Toxin.

15. Heterokonjugat zur Verwendung nach Anspruch 14 und Verwendung nach Anspruch 14, wobei die Verbindung ein Chelator ist, der ausgewählt ist aus der Gruppe bestehend aus DTPA, DOTA, TETA, NOTA und einem geeigneten Peptid, an das eine nachweisbare Markierung oder ein cytotoxisches Agens konjugiert sein kann.

16. Heterokonjugat zur Verwendung nach Anspruch 14 und Verwendung nach Anspruch 14, wobei die Verbindung ein chemotherapeutisches Agens ist, das ausgewählt ist aus der Gruppe bestehend aus Anthracyclinen, Taxanen, Stickstoffsenfgas, Ethylenimin-Derivaten, Alkylsulfonaten, Nitrosoharnstoffen, Triazenen, Folsäureanalogen, Pyrimidinanalogen, Purinanalogen, Antisinnoligonukleotiden, Antagonisten oder Inhibitoren von Transkriptionsfaktoren, Alkaloiden, Antibiotika, Enzymen, Platinkoordinationskomplexen, COX-2-Inhibitoren, apoptotischen Agenzien, Thalidomid und seinen Derivaten, substituiertem Harnstoff, Methylhydrazin-Derivaten und Nebennierenrinden-Suppressoren oder -Antagonisten.

## Revendications

1. Hétéroconjugué comprenant au moins un premier bras de liaison et un second bras de liaison,
dans lequel ledit premier bras de liaison comprend un anticorps monoclonal anti-CD20 ou un fragment de celui-ci se liant à un antigène,
dans lequel ledit second bras de liaison comprend un anticorps monoclonal anti-CD22 ou un fragment de celui-ci se liant à un antigène, et
dans lequel ledit hétéroconjugué est capable d'induire l'apoptose d'une cellule Daudi quand ledit hétéroconjugué est mis en contact avec la cellule Daudi pendant 24 heures,
pour son utilisation dans un procédé de traitement d'un lymphome associé aux lymphocytes B, d'une leucémie lymphoïde chronique ou d'une leucémie lymphoïde aiguë.

2. Utilisation d'un hétéroconjugué comprenant au moins un premier bras de liaison et un second bras de liaison,
dans lequel ledit premier bras de liaison comprend un anticorps monoclonal anti-CD20 ou un fragment de celui-ci se liant à un antigène,
dans lequel ledit second bras de liaison comprend un anticorps monoclonal anti-CD22 ou un fragment de celui-ci se liant à un antigène, et
dans lequel ledit hétéroconjugué est capable d'induire l'apoptose d'une cellule Daudi quand ledit hétéroconjugué est mis en contact avec la cellule Daudi pendant 24 heures,
pour la préparation d'un médicament destiné au traitement d'un lymphome associé aux lymphocytes B, d'une leucémie lymphoïde chronique ou d'une leucémie lymphoïde aiguë.

3. Hétéroconjugué pour son utilisation selon la revendication 1 et utilisation selon la revendication 2,
dans lequel ledit anticorps monoclonal anti-CD20 ou fragment de celui-ci se liant à un antigène comprend des CDR de la chaîne légère et de la chaîne lourde de l'anticorps hA20,
et
dans lequel ledit anticorps anti-CD22 ou fragment de celui-ci se liant à un antigène comprend des CDR de la chaîne légère et de la chaîne lourde de l'anticorps hLL-2.

4. Hétéroconjugué pour son utilisation selon la revendication 1 et utilisation selon la revendication 2,
dans lequel lesdits anticorps monoclonal anti-CD20 et anticorps monoclonal anti-CD22 comprennent respectivement des séquences de structure de régions variables de chaînes légères et lourdes d'un anticorps humain.

5. Hétéroconjugué pour son utilisation selon la revendication 1 et utilisation selon la revendication 2,
dans lequel lesdits premier et second bras de liaison ne présentent pas d'activité apoptotique quand ils ne sont pas conjugués l'un à l'autre.

6. Hétéroconjugué pour son utilisation selon la revendication 1 et utilisation selon la revendication 2,
dans lequel ledit hétéroconjugué comprend une protéine de fusion comprenant ledit premier et ledit second bras de liaison.

7. Hétéroconjugué pour son utilisation selon la revendication 1 et utilisation selon la revendication 2,
dans lequel ledit premier et ledit second bras de liaison sont conjugués par l'intermédiaire d'une liaison chimique.

8. Hétéroconjugué pour son utilisation selon la revendication 1 et utilisation selon la revendication 2,
dans lequel ledit hétéroconjugué induit l'apoptose d'une manière dose-dépendante sans réticulation des hétéroconjugués les uns avec les autres.

9. Hétéroconjugué pour son utilisation selon la revendication 1 et utilisation selon la revendication 2,
dans lequel ledit hétéroconjugué est un hétéroconjugué multispécifique qui se lie à plus de deux antigènes.

10. Hétéroconjugué pour son utilisation selon la revendication 1 et utilisation selon la revendication 2,
dans lequel lesdits bras de liaison sont des anticorps humains, murins, chimères, primatisés ou humanisés ou des fragments se liant à un antigène.

11. Hétéroconjugué pour son utilisation selon la revendication 1 et utilisation selon la revendication 2,
dans lequel ledit hétéroconjugué possède une structure choisie dans le groupe constitué par IgG x Fab', IgG x sFv, F(ab')₂ x Fab', Fab' x Fab', Fab' x sFv, (sFv x sFv)₂, sFv x sFv, un diabody, un triabody, un tétrabody et un quintabody.

12. Hétéroconjugué pour son utilisation selon la revendication 1 et utilisation selon la revendication 2,
dans lequel ledit hétéroconjugué est composé d'un anticorps bispécifique ou d'un anticorps multispécifique contenant plus de deux protéines à liaison spécifique.

13. Hétéroconjugué pour son utilisation selon la revendication 12 et utilisation selon la revendication 12,
dans lequel ledit anticorps bispécifique possède une structure Fab' X Fab' bivalente.

14. Hétéroconjugué pour son utilisation selon la revendication 1 et utilisation selon la revendication 2,
dans lequel ledit hétéroconjugué comprend en outre au moins un composé choisi dans le groupe constitué par un chélateur, un agent chimiothérapeutique, une enzyme, une hormone, un immunomodulateur, un oligonucléotide, un radionucléide, un composé boré, un agent photoactif et une toxine.

15. Hétéroconjugué pour son utilisation selon la revendication 14 et utilisation selon la revendication 14,
dans lequel ledit composé est un chélateur choisi dans le groupe constitué par le DTPA, le DOTA, le TETA, le NOTA et un peptide approprié auquel un marqueur détectable ou un agent cytotoxique peut être conjugué.

16. Hétéroconjugué pour son utilisation selon la revendication 14, et utilisation selon la revendication 14 dans lequel ledit composé est un agent chimiothérapeutique choisi dans le groupe constitué par les anthracyclines, les taxanes, les moutardes à l'azote, les dérivés d'éthylènimine, les sulfonates d'alkyle, les nitrosourées, les triazènes ; les analogues de l'acide folique, les analogues de pyrimidine, les analogues de purine, les oligonucléotides antisens, les antagonistes ou inhibiteurs de facteurs de transcription, les alcaloïdes, les antibiotiques, les enzymes, les complexes de coordination du platine, les inhibiteurs de COX-2, les agents apoptotiques, le thalidomide et ses dérivés, l'urée substituée, les dérivés de méthylhydrazine, et les anticortisoliques ou les antagonistes cortisoliques.
